# EUROPEAN PATENT SPECIFICATION

(11) **EP 0 717 777 B1**
(45) Date of publication and mention of the grant of the patent: **29.03.2006**
(21) Application number: 94923374.6
(22) Date of filing: 07.07.1994
(51) Int. Cl.: C12N 15/31, C12N 15/10, C12N 15/74, C12N 1/21, C07K 14/255, C12Q 1/68, A61K 39/112, C12R 1/42

(54) **SALMONELLA VACCINES**
SALMONELLA IMPFSTOFFE
VACCINS A SALMONELLE

(30) Priority: 09.07.1993 US 90526; 06.07.1994 US 271354
(43) Date of publication of application: 26.06.1996
(73) Proprietor: THE GENERAL HOSPITAL CORPORATION, Boston, MA 02114 (US); PRESIDENT AND FELLOWS OF HARVARD COLLEGE, Cambridge, MA 02138-5701 (US)
(72) Inventor: MILLER, Samuel I., III, Brookline, MA 02146 (US); MEKALANOS, John J., Cambridge, MA 02138 (US)
(74) Representative: Webber, Philip Michael
(86) International application number: PCT/US1994/007658
(87) International publication number: WO 1995/002048

(56) References cited:
- WO-A-92/11361
- MILLER S.I. ET AL.: "A two-component regulatory system (PhoP PhoQ) control S. typhimurium virulence" PROC. NATL. ACAD. SCI. USA, vol. 86, July 1989, pages 5054-5058, XP002060845
- VACCINE, Vol. 11, No. 2, issued 1993, MILLER et al., "The PhoP Virulence Regulon and Live Oral Salmonella Vaccines", pages 122-125.
- MOLECULAR MICROBIOLOGY, Vol. 5, No. 9, issued 1991, MILLER, "PhoP/PhoQ: Macrophage-Specific Modulators of Salmonella Virulence ?", pages 2073-2078.
- RESEARCH MICROBIOLOGY, Vol. 141, issued 1990, MILLER et al., "Salmonella Vaccines with Mutations in the phoP Virulence Regulon", pages 817-821.

## Description

### Background of the Invention

The invention relates to vaccines.

This invention was made with Government support under Grant No. AI30479 and Grant No. 00917 awarded by the National Institutes of Health. The Government has certain rights in the invention.

Enteric fevers and diarrheal diseases, e.g., typhoid fever and cholera, are major causes of morbidity and mortality throughout the developing world, Hook et al., 1980, In Harrison's Principles of Internal Medicine, 9th Ed., 641-848, McGraw Hill, New York. Traditional approaches to the development of vaccines for bacterial diseases include the parenteral injection of purified components or killed organisms. These parenterally administered vaccines require technologically advanced preparation, are relatively expensive, and are often, because of dislike for needle-based injections, resisted by patients. Live oral vaccine strains have several advantages over parenteral vaccines: low cost, ease of administration, and simple preparation.

The development of live vaccines has often been limited by a lack of understanding of the pathogenesis of the disease of interest on a molecular level. Candidate live vaccine strains require nonrevertable genetic alterations that affect the virulence of the organism, but not its induction of an immune response. Work defining the mechanisms of toxigenesis of *Vibrio cholerae* has made it possible to create live vaccine strains based on deletion of the toxin genes, Mekalanos et al., 1983, Nature 306:551, Levine et al., 1988, Infect. Immun. 56:161.

Recent studies have begun to define the molecular basis of *Salmonella typhimurium* macrophage survival and virulence, Miller et al., 1989, Proc. Natl. Acad. Sci. USA 86:5054. *Salmonella typhimurium* strains with mutations in the positive regulatory regulon *pho*P are markedly attenuated in virulence for BALB/c mice. The *phoP* regulon is composed of two genes present in an operon, termed *phoP* and *phoQ.* The *phoP* and *phoQ* gene products are highly similar to other members of bacterial two-component transcriptional regulators that respond to environmental stimuli and control the expression of a large number of other genes. A mutation at one of these *phoP* regulatory region regulated genes, *pagc*, confers a virulence defect. Strains with *pag*c, *phoP*, or *phoQ* mutations afford partial protection to subsequent challenge by wild-type *S*. *typhimurium.*

*Salmonella* species cause a spectrum of clinical disease that includes enteric fevers and acute gastroenteritis., Hook et al., 1980, *supra*. Infections with *Salmonella* species are more common in immunosuppressed persons, Celum et al., 1987, J. Infect. Dis. 156:998. *S. typhi,* the bacterium that causes typhoid fever, can only infect man, Hook et al., 1980, *supra.* The narrow host specificity of *S. typhi* has resulted in the extensive use of *S. enteriditis typhimurium* infection of mice as a laboratory model of typhoid fever, Carter et al., 1984 J. Exp. Med. 139:1189. *S. typhimurium* infects a wider range of hosts, causing acute gastroenteritis in man and a disease similar to typhoid fever in the mouse and cow.

*Salmonella* infections are acquired by oral ingestion. The organisms, after traversing the stomach, replicate in the small bowel, Hornik et al., 1970, N. Eng. J. Med. 283:686. *Salmonella* are capable of invasion of the intestinal mucosal cells, and *S. typhi* can pass through this mucosal barrier and spread via the Peyer's patches to the lamina propria and regional lymph nodes. Colonization of the reticuloendothelial cells of the host then occurs after bacteremia. The ability of *S. typhi* to survive and replicate within the cells of the human reticuloendothelial system is essential to its pathogenesis, Hook et al., 1980, *supra*, Hornick et al., 1970, *supra*, and Carter et al., 1984, *supra*.

Immunity to *Salmonella typhi* involves humoral and cell-mediated immunity, Murphy et al., 1987, J*.* Infect. Dis. 156:1005, and is obtainable by vaccination, Edelman et al., 1986, Rev. Inf. Dis. 8:324. Recently, human field trials demonstrated significant protective efficacy against *S. typhi* infection after intramuscular vaccination with partially purified Vi antigen, Lanata et al., 1983, Lancet 2:441. Antibody-dependent enhancement of *S*. *typhi* killing by T cells has been demonstrated in individuals who received a live *S. typhi* vaccine, indicating that these antibodies may be necessary for the host to generate a cell-mediated immune response, Levine et al., 1987, J. Clin. Invest. 79: 888. The cell-mediated immune response is important in typhoid immunity since killed vaccines that do not induce this immune response are not protective in man, Collins et al., 1972, Infect. Immun. 41 :742.

### Summary of the Invention

The invention provides a *Salmonella* vaccine which does not cause transient bacteremia. In general, the invention features a Salmonella cell, e.g., a *S. typhi*, *S. enteritidis*, *S. Typhimurium,* or *S*. *Cholerae-suis* cell, the virulence of which is attenuated by a mutation in a PhoP regulon. As used herein, PhoP regulon is defined as a DNA which comprises a unit of salmonella virulence gene expression characterized by two regulatory genes, *phoP* and *phoQ,* and structural genes, the expression of which is regulated by *phoP* and *phoQ,* e.g., phoP regulatory region repressed genes (prg) or phoP regulatory region activated genes (pag). Such a bacterial cell can be used as a vaccine to immunize a mammal against salmonellosis.

The *Salmonella* cell may be of any serotype, e.g., *S*. *typhimurium*, *S. paratyphi A, S. paratyphi B, S. paratyphi C, S. pylorum, S. dublin, S. heidelberg, S. newport*, *S. minnesota*, *S. Infantis, S. virchow,* or *S. panama*.

The mutation is a non-revertable null mutation in the PhoP/PhoQ locus, which comprises a deletion of nucleotides 376 to 1322 of the *PhoP*/*PhoQ* regulatory locus.

An optional second mutation may be a non-revertable null mutation in an *aro*A locus or a non-revertable null mutation in an *aro*C/*aro*D locus, or another locus involved in the biosynthesis of aromatic amino acids.

To further attenuate the virulence of the bacterial cell of the invention, the cell may contain yet another mutation, e.g., a deletion, in a non-aromatic amino acid synthetic gene, e.g., a mutation which renders the cell auxotrophic for a non-aromatic amino acid, e.g., histidine. In preferred embodiments, the bacterial cell of the invention is a *S. typhi* cell with the genotype. ArOA⁻, His⁻, PhoP/PhoQ⁻, e.g., TyLH445.

Also disclosed is a *Salmonella* cell, the virulence of which is attenuated by the constitutive expression of a gene under the control of a two-component regulatory system. In preferred embodiments of that cell the constitutive expression is the result of a mutation at a component of the two-component regulatory system. In preferred embodiments the bacterial cell includes a second mutation which attenuates virulence.

Also disclosed is a vaccine wherein the two-component regulatory system is the phoP regulatory region, and the gene under the control of the two-component system is a phoP regulatory region regulated gene, e.g., a *prg* gene, e.g., *pryA, pryB, prgC, prgE,* or *prgH,* or *pag* gene, e.g., *pagC.* Constitutive expression may be the result of a change or mutation, e.g., a deletion, (preferably a non-revertible mutation) at the promoter of the regulated gene or of the phoP regulatory region, e.g., a mutation in the *phoQ* or the *phoP* gene, e.g., the Phop^{c} mutation.

The specification also discloses a vaccine including a bacterial cell which is attenuated by decrease of expression of a virulence gene under control of a *phoP* regulatory region, e.g., a *prg* gene, e.g., *prgA, prgB, PrgC, prgE*, or *PrgH.*

Also disclosed is a vaccine wherein the *Salmonella* cell includes a first mutation, e.g., a deletion, which attenuates virulence, e.g., a mutation in a *phoP* regulatory region gene, e.g., a mutation in the *phoP* or *pho*Q gene, e.g., PhoP^{c}, or a mutation in a phoP regulatory region regulated gene, and a second mutation which attenuates virulence, e.g., a mutation in an aromatic amino acid synthetic gene, e.g., an aro gene, a mutation in a phoP regulatory region regulated gene, e.g., a mutation in a *prg* gene, e.g., *prgA, prgB, prgC*, *prgE,* or *prgH,* or *pag* locus, e.g., a *pagC* mutation.

Also disclosed is a bacterial cell which include a first mutation in a phoP regulatory region gene and a second mutation in an aromatic amino acid synthetic gene, e.g, an aro gene.

In another aspect, the invention features a vaccine comprising a live *Salmonella* cell the virulence of which is attenuated by a non-revertible null mutation within the *phoP*/*phoQ* locus, such that the cell genotype is *phoP*/*phoQ,*^{*-*}*wherein* said non-revertible null mutation comprises a deletion of nucleotides 376-1322 within the *phoP*/*phoQ* locus. In preferred embodiments the salmonella cell includes a virulence attenuating mutation in a second gene, e.g., in an aromatic amino acid synthetic gene, e.g., an aro gene.

Also disclosed is a vaccine wherein the bacterial cell is a *Salmonella* cell, the two-component regulatory system is the phoP regulatory region, and the gene under its control is a *prg* gene, e.g. *prgrA*, *prgB, prgC, prgE,* or *prgH,* or a *pag* gene, e.g., the *pagC* gene.

Also disclosed is a vaccine, preferably a live vaccine, including a *Salmonella* cell e.g., a *S*. *typhi*, *S. enteritidis typhimurium,* or *S*. *cholerae-suis* cell, including a first virulence attenuating mutation in an aromatic amino acid biosynthetic gene, e.g., an aro gene, and a second virulence attenuating mutation in a phoP regulatory region gene, e.g., a *phoP*^{*-*} mutation.

Also disclosed is a live *Salmonella* cell, or a substantially purified preparation thereof, e.g., a *S*. *typhi*, *S. enteriditis typhimurium,* or *S. cholerae-suis* cell, in which there is inserted into a virulence gene, e.g., a gene in the phoP regulating region, or a phoP regulating region regulated gene, e.g., a *prg* gene, e.g., *prgA, prgB, prgC, prgB,* or *prgH* or a *pag* locus, e.g., *pag*C, a gene encoding a heterologous protein, or a regulatory element thereof.

In preferred embodiments the live *Salmonella* cell carries a second mutation, e.g., an aro mutation, e.g., an aroA mutation, e.g., aroA⁻ or aroADEL407, that attenuates virulence.

The DNA encoding a heterologous protein may be under the control of an environmentally regulated promoter. The live *Salmonella* cell may further include a DNA sequence encoding T7 polymerase under the control of an environmentally regulated promoter and a T7 transcriptionally sensitive promoter, the T7 transcriptionally sensitive promoter controlling the expression of the heterologous antigen.

Also disclosed is a vector capable of integrating into the chromosome of *Salmonella* including: a first DNA sequence encoding a heterologous protein; a second (optional) DNA sequence encoding a marker e.g., a selective marker, *e*.*g*., a gene that confers resistance for a heavy metal resistance or a gene that complements an auxotrophic mutation carried by the strain to be transformed; and a third DNA sequence, e.g., a *pho*P regulon encoded gene, e.g., a *prg* gene, e.g., *prg*A, *prg*B, *prg*C, *prgE,* or *prgH* or a *pag* locus, e.g., *pag*C, encoding a *pho*P regulatory region regulated gene product necessary for virulence, the third DNA sequence being mutationally inactivated.

In other disclosed examples: The first DNA sequence may be disposed on the vector so as to mutatitsnally inactivate the third DNA sequence; the vector cannot replicate in a wild-type *Salmonella* strain; the heterologous protein is under the control of an environmentally regulated promoter; and the vector further includes a DNA sequence encoding T7 polymerase under the control of an environmentally regulated promoter and a T7 transcriptionally sensitive promoter, the T7 transcriptionally sensitive promoter controlling the expression of the heterologous antigen.

The specification also discloses a method of vaccinating an animal, e.g., a mammal, e.g., a human, against a disease caused by a bacterium, e.g., *Salmonella,* including administering a vaccine of the invention.

Two-component regulatory system, as used herein, refers to a bacterial regulatory system that controls the expression of multiple proteins in response to environmental signals. The two-components referred to in the term are a sensor, which may, e.g., sense an environmental parameter and in response thereto promote the activation, e.g. by promoting the phosphorylation, of the second component, the activator. The activator affects the expression of genes under the control of the two-component system. A two-component system can include, e.g., a histidine protein kinase and a phosphorylated response regulator, as is seen in both gram positive and gram negative bacteria. In *E*. *coli*, e.g., 10 kinases and 11 response regulators have been identified. They control chemotaxis, nitrogen regulation, phosphate regulation, osmoregulation, sporulation, and many other cellular functions, Stock et al., 1989 Microbiol. Rev. 53:450-490, hereby incorporated by reference. A two-component system also controls the virulence of *Agrobacterium tumefasciens* plant tumor formation, Leroux et al. EMBO J 6:849-856. Similar virulence regulators are involved in the virulence of *Bordetella pertussis* Arico et al., 1989, Proc. Hatl. Acad. Sci. USA 86:6671-6675, hereby incorporated by reference, and *Shigella flexneri,* Bernardini et al., 1990, J. Bact. 172 :6274 - 6281.

Environmentally regulated, as used herein refers to a pattern of expression wherein the expression of a gene in a cell depends on the levels of some characteristic or component of the environment in which the cell resides. Examples include promoters in biosynthetic pathways which are turned on or off by the level of a specific component or components, e.g., iron, temperature responsive promoters, or promoters which are expressed more actively in specific cellular compartments, e.g., in macrophages or vacuoles.

A vaccine, as used herein, is a preparation including materials that evoke a desired biological response, e.g., an immune response, in combination with a suitable carrier. The vaccine includes a live organism, in which case it is usually administered orally. (Killed organisms or components thereof are usually administered parenterally.) The cells used for the vaccine of the invention are alive and thus capable of colonizing the intestines of the inoculated animal.

A mutation, as used herein, is any change (in comparison with the appropriate parental strain) in the DNA sequence of an organism. These changes can arise e.g., spontaneously, by chemical, energy e.g., X-ray, or other forms of mutagenesis, by genetic engineering, or as a result of mating or other forms of exchange of genetic information. Mutations include e.g., base changes, deletions, insertions, inversions, translocations or duplications.

A mutation attenuates virulence if, as a result of the mutation, the level of virulence of the mutant cell is decreased in comparison with the level in a cell of the parental strain, as measured by (a) a significant (e.g., at least 50%) decrease in virulence in the mutant strain compared to the parental strain, or (b) a significant (e.g., at least 50%) decrease in the amount of the polypeptide identified as the virulence factor in the mutant strain compared to the parental strain.

A non-revertible mutation, as used herein, is a mutation which cannot revert by a single base pair change, e.g., deletion or insertion mutations and mutations that include more than one lesion, e.g., a mutation composed of two separate point mutations.

The phoP regulatory region, as used herein, is a two-component regulatory system that controls the expression of *pag* and *prg* genes. It includes the phoP locus and the phoQ locus.

phoP regulatory region regulated genes, as used herein, refer to genes such as *pag* and *prg* genes.

*pag,* as used herein, refers to a gene which is positively regulated by the phoP regulatory region.

*prg,* as used herein, refers to a gene which is negatively regulated by the phoP regulatory region.

An aromatic amino acid synthetic gene, as used herein, is a gene which encodes an enzyme which catalyzes a step in the synthesis of an aromatic amino acid. *aro*A, *aro*C, and *aro*D are examples of such genes in *Salmonella.* Mutations in these genes can attenuate virulence without the total loss of immunogenicity.

Abnormal expressions, as used herein, means expression which is higher or lower than that seen in wild type.

Heterologous protein, as used herein, is a protein that in wild type, is not expressed or is expressed from a different chromosomal site, e.g., a heterologous protein is one encoded by a gene that has been inserted into a second gene.

Virulence gene, as used herein, is a gene the inactivation of which results in a *Salmonella* cell with less virulence than that of a similar *Salmonella* cell in which the gene is not inactivated. Examples include the phoP, *pag*C*, prgH* genes.

A marker, as used herein, is gene product the presence of which is easily determined, e.g., a gene product that confers resistance to a heavy metal or a gene product which allows or inhibits growth under a given set of conditions.

Purified preparation, as used herein, is a preparation, e.g., of a protein, which is purified from the proteins, lipids, and other material with which it is associated. The preparation is preferably at least 2-10 fold purified.

Constitutive expression, as used herein, refers to gene expression which is modulated or regulated to a lesser extent than the expression of the same gene in an appropriate control strain, e.g., a parental or in wild-type strain. For example, if a gene is normally repressed under a first set of conditions and derepressed under a second set of conditions constitutive expression would be expression at the same level, e.g., the repressed level, the derepressed level, or an intermediate level, regardless of conditions. Partial constitutive expression is included within the definition of constitutive expression and occurs when the difference between two levels of expression is reduced in comparison in what is seen in an appropriate control strain, e.g., a wild-type or parental strain.

A substantially purified preparation of a bacterial cell is a preparation of cells wherein contaminating cells without the desired mutant genotype constitute less than 10%, preferably less than 1%, and more preferably less than 0.1% of the total number of cells in the preparation.

The invention allows for the attenuation of virulence of bacteria and of vaccines that include live bacteria, by mutations in two-component regulatory systems. The vaccines of the invention are highly attenuated for virulence but retain immunogenicity, thus they are both safe and effective. The vectors disclosed herein allow the rapid construction of strains containing DNA encoding heterologous proteins, e.g., antigens. The heterologous protein encoding DNA is chromosomally integrated, and thus stable, unlike plasmid systems which are dependent on antibiotic resistance or other selection pressure for stability. Live salmonella cells in which the expression of heterologous protein is under the control of an environmentally responsive promoter do not express the heterologous protein at times when such expression would be undesirable e.g., during culture, vaccine preparation, or storage, contributing to the viability of the cells, but when administered to humans or animals, express large amounts of the protein. This is desirable because high expression of many heterologous proteins in *Salmonella* can be associated with toxicity to the bacterium. The use of only a single integrated copy of the DNA encoding the heterologous protein also contributes to minimal expression of the heterologous protein at times when expression is not desired. In embodiments where a virulence gene, e.g., the *pagC* gene or the *prgH* gene, contains the site of integration for the DNA encoding the heterologous protein the virulence of the organism is attenuated.

A substantially pure DNA, as used herein, refers to a nucleic acid sequence, segment, or fragment, which has been purified from the sequences which flank it in a naturally occurring state, e.g., a DNA which has been removed from the sequences which are normally adjacent to the fragment, e.g., the sequences adjacent to the fragment in the genome in which it naturally occurs. The term also applies to DNA which has been substantially purified from other components which naturally accompany the DNA, e.g., DNA which has been purified from proteins which naturally accompany it in a cell.

Other features and advantages of the invention will be apparent from the following description of the preferred embodiments and from the claims.

### Description of the Preferred Embodiments

The drawings will first be described.

### Drawings

Fig. 1 is a graph of the survival of *Salmonella* strains within macrophages.
Fig. 2 is a map of the restriction endonuclease sites of the *pag*C locus.
Fig. 3 is a map of the DNA sequence of the *pag* C region (SEQ ID NO:1).

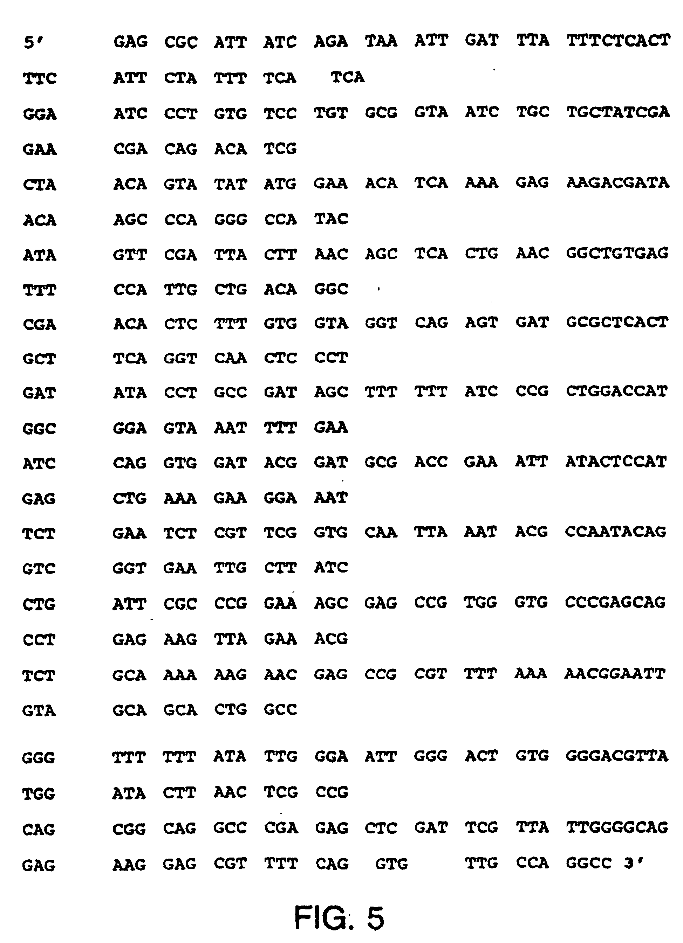

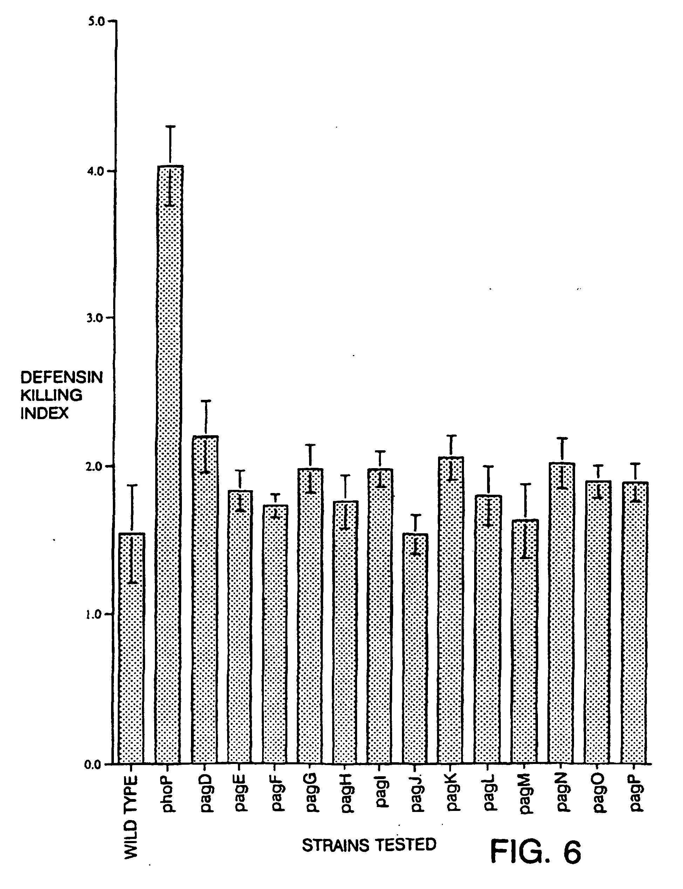

Fig. 4 is a map of the location of *prgH* within the *hil* locus. The arrows indicate the direction of orientation of the neomycin promoter of *Tn5*B50 insertions within the *hil* locus and the direction of transcription of the *prgH1::*Tn*phoA* fusion protein. Restriction endonuclease sites are represented by B, *Bam*H1; H, *Hind*III; X, *Xho*I; *S, Sac*I; V, *Eco*RV.
Fig. 5 is a DNA sequence from the *prg*H gene (plasmid pIB01) (SEQ ID NO:3).
Fig. 6 is a bar graph showing a comparison of the sensitivity of wild type (ATCC 14028), PhoP-null mutant (CS015), and *pag*::Tn*phoA* mutant strains to NP-1 defensin. The y-axis represents the Defensin Killing Index (DKI) which is a measure of bacteria killed on exposure to NP-1. The DKI is defined as the logarithmic function of the ratio of control bacteria to surviving bacteria incubated with NP-1 [DKI=log (CFU without NP-1/CFU with NP-1)]. The individual bars represent the mean and standard error of five separate experiments. The x-axis indicates the allele mutated. The mean DKI for each of the *pag*::Tn*phoA* strains tested was determined not be different from that of wild type *Salmonella.* (P<0.05). In contrast, the *phoP* mutant was significantly different (P<0.0001).

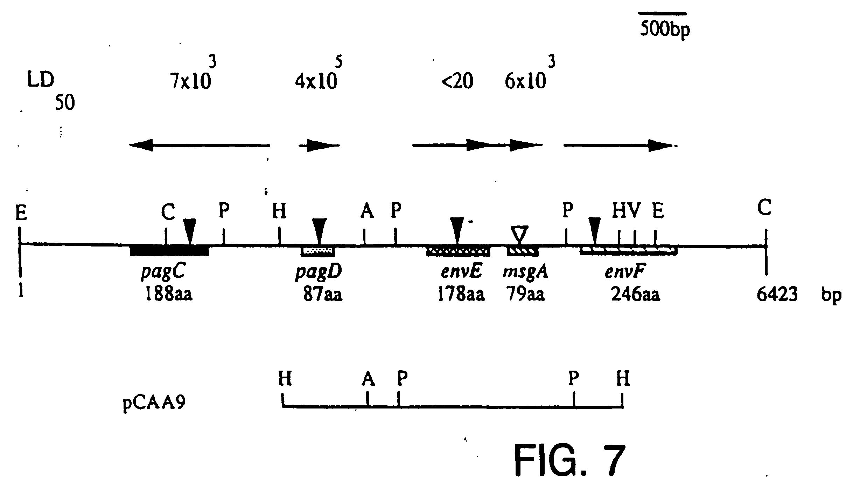

Fig. 7 is a diagram showing a partial physical map of the restriction endonuclease sites of the *pagC* chromosomal region. The mouse 50% lethal doses (LD₅₀) for strains with transposon insertions in *pagD, envE, msgA*, and *pagC* are shown above each gene. Horizontal arrows demonstrate the direction of transcription. Vertical arrows denote Tn*phoA* insertions and the hollow triangle denotes a MudJ insertion. Below the chromosomal map is a representation of the DNA insert in plasmid pCAA9, which was mutagenized with Tn*phoA* and MudJ. Letter designations: A, *Acc*I; C, *Cla*I; E, *Eco*RI; H, *Hpa*I; P, *Pst*I; and V, *Eco*RV.

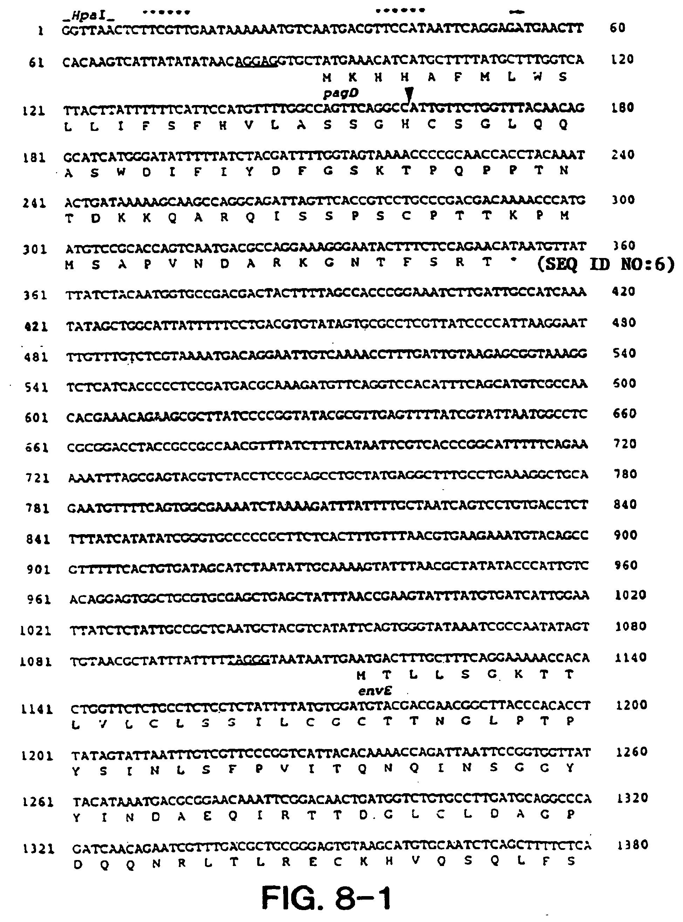

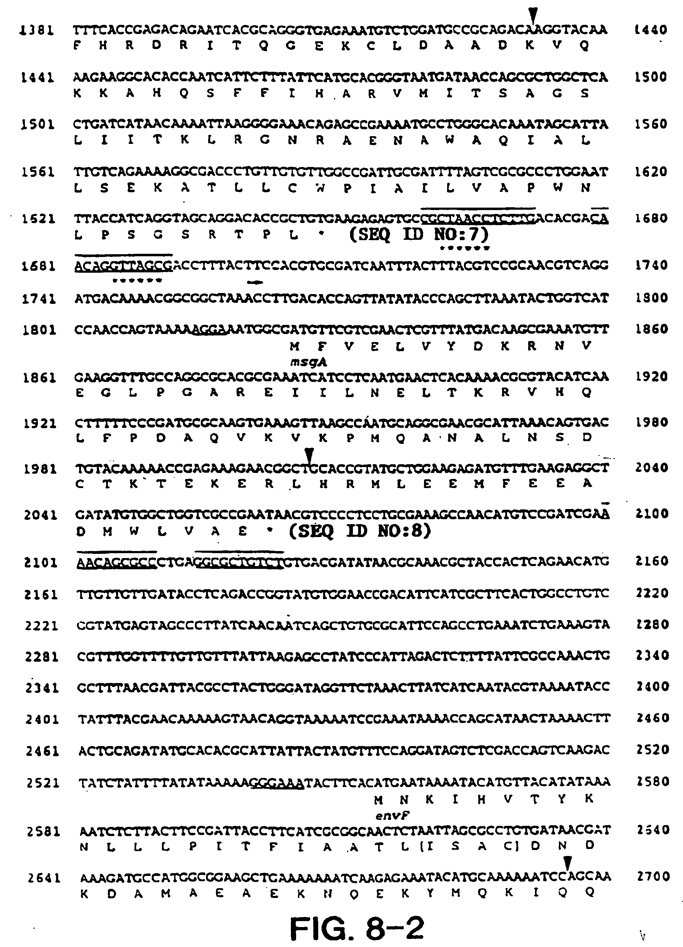

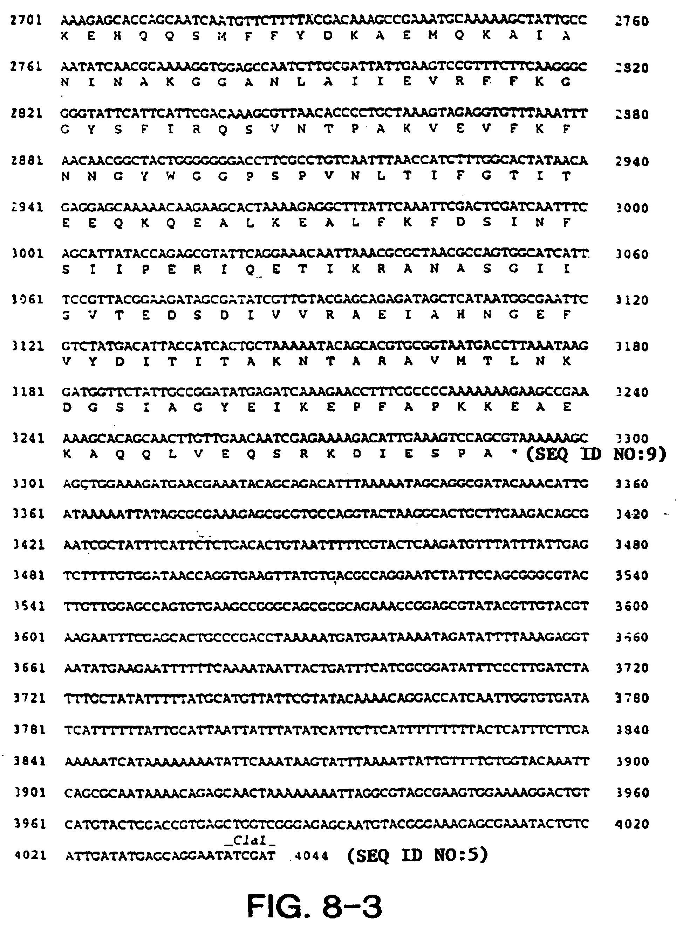

Fig. 8 is a DNA sequence of the region upstream of *pagC* and the translation of each ORF. The *H*paI and *Cla*I sites at the beginning and end of the region are indicated. Shine-Delgarno regions are underlined and stem loop structures (potential Rho-independent terminators) are indicated with a line below and above the sequence. Arrow heads denote the location of the representative transposon insertion within each gene. Horizontal arrows in the *pagD* and *msgA* promoter regions mark the transcriptional start sites, and asterisks mark the -10 and -35 sequences. The consensus lipid attachment site in EnvF is enclosed in brackets. The *pagD* ORF begins at nucleotide 91 and ends at nucleotide 354 of SEQ ID NO:5; the *envE* ORF begins at nucleotide 1114 and ends at nucleotide 1650 of SEQ ID NO:5; the *msgA* ORF begins at nucleotid 1825 and ends at nucleotide 2064 of SEQ ID NO:5; and the envF ORF begins at nucleotide 2554 and ends at nucleotide 3294 of SEQ ID NO:5.
Fig. 9 is a DNA sequence containing the *prgH, prgI, prgJ,* and *prgK* genes. The start codon (ATG) of each gene is underlined, and the stop codon is indicated with an asterisk. The *prgH* ORF begins at nucleotide 688 and ends at 1866 of SEQ ID NO:10; the *prgI* ORF begins at nucleotide 1891 and ends at nucleotide 2133 of SEQ ID NO:10; the *prgJ* ORF begins at nucleotide 2152 and ends at nucleotide 2457 of SEQ ID NO:10; and the *prgK* ORF begins at nucleotide 2454 and ends at nucleotide 3212 of SEQ ID NO:10.
Fig. 10 is a line graph showing the growth rates of the parent *Salmonella* strain (AroA-) and the vaccine strain (AroA-, PhoP-).
Fig. 11 is a bar graph showing defensin sensitivity of mouse vaccine strains (*S. typhimurium*).
Fig. 12 is a bar graph showing phoP activation as measured by LacZ activity using the PagB:LacZ recorder fusion construct.
Fig. 13 is a bar graph showing defensin sensitivity of *S*. *typhi* vaccine strain TyLH445 compared to the AroA⁻ parent strain.
Fig. 14A is a graph showing the relative expression of constitutive expression (610 and 617) and phoP regulated (PagC and pagD) expression of AP fusion proteins.
Fig. 14B is a graph showing the immune response to lipopolysaccharide (LPS).
Fig. 14C is a graph showing the immune response to the model heterologous antigen, AP.

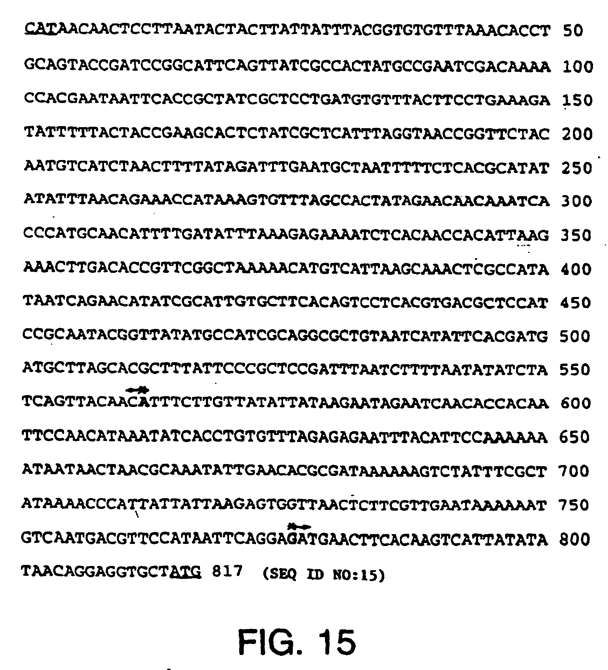

Fig. 15 is a DNA sequence containing the *pagC-pagD* intergenic region. *pagC* translational start site (ATG on the opposite DNA strand) is underlined (nucleotides 1-3 of SEQ ID NO:15). The *pagC* transcriptional start (nucleotide 562) is indicated with an arrow pointing left. The *pagD* translational start (ATG) is underlined (nucleotides 815-817 of SEQ ID NO:15). The *pagD* transcriptional start is indicated with an arrow pointing right (nucleotide 776).

### Strain Deposit

Under the terms of the Budapest Treaty on the International Recognition of the Deposit of Microorganisms for the Purpose of Patent Procedure, deposit of the following materials has been made with the American Type Culture Collection (ATCC) of Rockville, MD, USA.

Applicant's assignee, Massachusetts General Hospital, represents that the ATCC is a depository affording permanence of the deposit and ready accessibility thereto by the public if a patent is granted. All restrictions on the availability to the public of the material so deposited will be irrevocably removed upon the granting of a patent. The material will be available during the pendency of the patent application to one determined by the Commissioner to be entitled thereto under 37 CFR 1.14 and 35 U.S.C. S122. The deposited material will be maintained with all the care necessary to keep it viable and uncontaminated for a period of at least five years after the most recent request for the furnishing of a sample of the deposited plasmid, and in any case, for a period of at least thirty (30) years after the date of deposit or for the enforceable life of the patent, whichever period is longer. Applicants' assignee acknowledges its duty to replace the deposit should the depository be unable to furnish a sample when requested due to the condition of the deposit.

*PhoP*^{*c*} strain CS022 (described below) has been deposited with the American Type Culture Collection (Roctcville, MD) and has received ATCC designation 55130.

The plasmid, pIB01, containing the *prgH* gene has been deposited on July 9, 1993 with the American Type Culture Collection (Rockville, MD) and has received ATCC designation ATCC 75496.

| Table 1: Bacterial strains and properties | | | |
|---|---|---|---|
| Strain | Genotype | Enzyme activity (U)^{a} | Reference or source |
| 10428 | Wild type | 180 (A) | ATCC; Miller et al., 1989, supra |
| TA2367 | *pho-24* | 1,925 (A) | Kier et al., 1974, supra |
| CS003 | Δ*phoP* Δ*purB* | <10 (A) | Miller et al., 1989, supra |
| CS022 | *pho-24* | 1,750 (A) | This work |
| CS023 | *pho-24 phoN2 zxx*::6251Tn10d-Cam | 25 (A) | This work |
| CS012 | *pag*A1::MU dJ | 45 (B) | Miller et al., 1989, supra |
| CS013 CS119 | *pag*B1::MU dJ *pag*C1::Tn*phoA phoN2 zxx*::6251Tn*10*d-Cam | 120 (B) 85 (C) | Miller et al., 1989, supra Miller et al., 1989, supra |
| SC024 | *pag*A1::Mu dJ *pho-24* | 450 (B) | This work |
| SC025 | *pag*B1::Mu dJ *pho-24* | 980 (B) | This work |
| SC026 | *pag*C1::Tn*phoApho-24phoN2* zxx::6251Tn*10*d-Cam | 385 (B) | This work |
| CS015 | *phoP102*::Tn*10*d-Cam | <10 (A) | Miller et al., 1989, supra |
| TT13208 | *phoP105::*Tn*10d* | <10 (A) --^{b} | |
| ^{a} | A. Acid phosphatase; B, β-galactosidase; C, alkaline phosphatase (AP). | | |
| ^{b} | Gift of Ning Zhu and John Roth. | | |

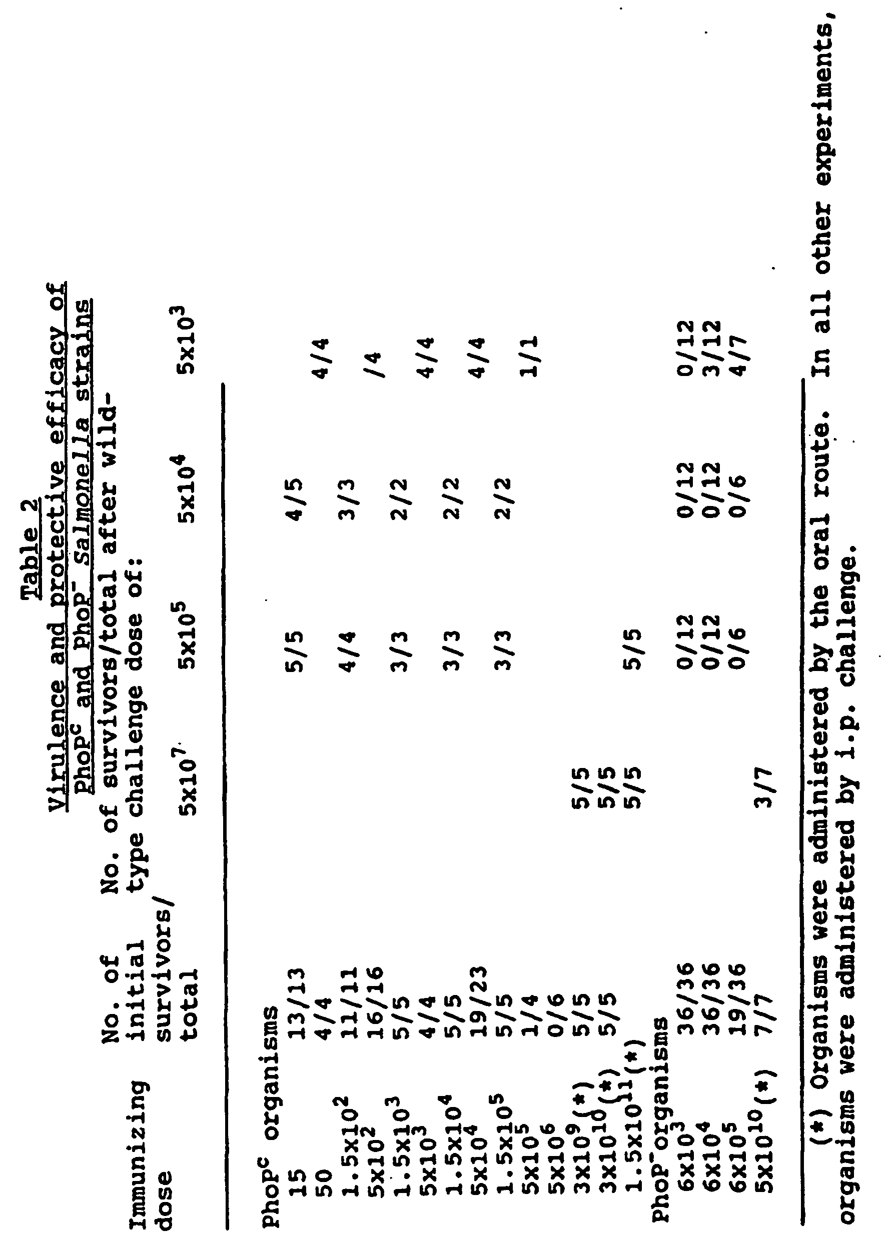

### The PhoP^{c} strain is deficient in survival within macrophages

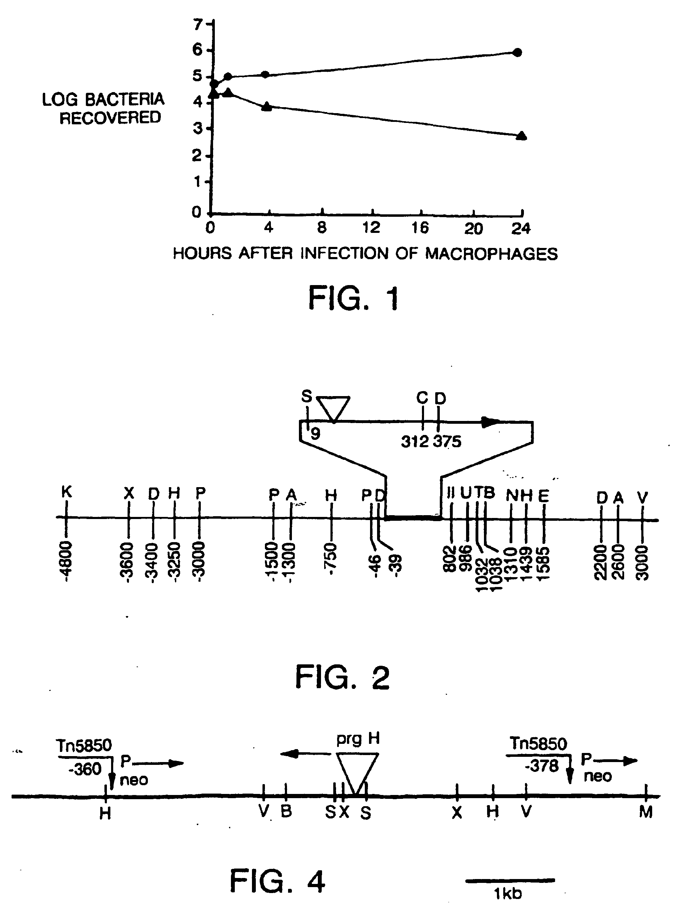

Because of the importance of survival within macrophages to *Salmonella* virulence Fields et al., 1986, Proc. Natl. Acad. Sci. USA 83:5189, PhoP^{c} bacteria were tested for this property. Strain CS022 was defective in the ability to grow and persist in macrophages as compared with wild-type organisms (Fig. 1). In Fig. 1 the survival of strain CS022 (PhoP^{c}) (triangles) in cultured macrophages is compared with that of wild-type *S*. *typhimurium* ATCC 10428 (cicles). The experiment shown is a representative one. The difference between the two strains at 4 and 24 hours is significant (P < 0.05). PhoP- bacteria seemed to have a macrophage survival defect qualitatively similar to that of PhoP^{c} bacteria but survived consistently better by two- to threefold in side-by-side experiments. The increased recovery of organisms that reverted to PhoP^{c} phenotype in mouse organs rich in macrophage content is consistent with the reduced macrophage survival of PhoP^{c} mutants in vitro.

### Use of the PhoP^{c} strain as a live vaccine

It has been previously reported that PhoP⁻ strains are useful as live vaccines in protecting against mouse typhoid, Miller et al., 1989, supra. The immunogenicity of PhoP^{c} when used as live attenuated vaccines in mice was compared with the of PhoP⁻. This was done by simultaneous determination of survival, after graded challenge doses with the wild-type strain ATCC 10428, in mice previously immunized with graded doses of the two live vaccine strains. CS015 *phoP*::Tn*10*d-Cam and CS022 *pho-24,* as well as a saline control. The results obtained (Table 2) suggest the following conclusions: (i) small i.p. doses of the PhoP^{c} strain (e.g., 15 organisms) effectively protect mice from challenge doses as large as 5x10⁵ bacteria (a challenge dose that represents greater than 10⁴ i.p. LD₅₀S), (ii) large doses of PhoP^{c} organisms given orally completely protect mice from an oral challenge consisting of 5x10⁷ wild-type bacteria (over 200 oral wild-type LD₅₀s) and (iii) by comparison, a large dose of PhoP⁻ organisms (5x10⁵) does not provide similar protection. The reversion of the PhoP^{c} mutation in vivo somewhat complicates the analysis of the use of these strains as vaccines, since revertants of the CS022 strain (i.e., wild-type cells) could increase immunogenicity). However, we were unable to identify revertants by examining 10% of the available spleen and liver tissue from those mice that received 10⁴ or fewer organisms.

### Strains, Materials and Methods

The strains, materials, and methods used in the PhoP regulon work described above are as follows.

American Type Culture Collection (ATCC) strain 14028, a smooth virulent strain of *S. typhimurium*, was the parent strain for all virulence studies. Strain TT13208 was a gift from Nang Zhu and John Roth. Strain TA2367 was a generous gift of Gigi Stortz and Bruce Ames, Kier et al., 1979, supra. Bacteriophage P22HT int was used in transductional crosses to construct strains isogenic except for *phoP* locus mutations, Davis et al., 1980, Advanced Bacterial Genetics, p. 78, 87. Cold Spring Harbor Laboratory, Cold Spring Harbor, NY. Luria broth was used as rich medium, and minimal medium was M9, Davis et al., 1980, supra. The chromogenic phosphatase substrate 5-bromo-4-chloro-3indolyl phosphate (XP) was used to qualitatively access acid and AP production in solid media.

Derivatives of *S. typhimurium* ATCC 10428 with the *pho-24* mutation were constructed by use of strain TA2367 as a donor of the *purB* gene in a P22 transductional cross with strain CS003 Δ*phoP* Δ*purB,* Miller et al., 1989, supra. Colonies were then selected for the ability to grow on minimal medium. A transductant designated CS022 (phenotype PhoP^{c}) that synthesized 1,750 U of acid phosphatase in rich medium (a ninefold increase over the wild-type level in rich medium) was used in further studies.

Derivatives of strains CS022 and CS023 *pho-24 phoN2 zxx*::6251Tn*10*d-Cam, and acid phosphatase-negative derivative of CS022, containing *pag* gene fusions were constructed by bacteriophage P22 transductional crosses, using selection of *TnphoA*- or Mu dJ-encoded kanamycin resistance. Strains were checked for the intact *pag* gene fusion by demonstration of appropriate loss of fusion protein activity on introduction of a *pho*P*105*::Tn*10*d or *pho*P*102*::Tn*10*d-Cam allele.

Assays of acid phosphatase, AP, and β-galactosidase were performed as previously described, Miller et al., 1989, supra and are reported in units as defined in Miller, 1972, Experiments in molecular genetics, p. 352-355, Cold Spring Harbor Laboratory, Cold Spring Harbor, NY.

In the mouse virulence and vaccination studies bacteria grown overnight in Luria broth were washed and diluted in normal saline. The wild-type parent strain of CS022 (ATCC 10428) was used for all live vaccine challenge studies. This strain has a 50% lethal dose (LiD₅₀) for naive adult BALB/c mice of less than 20 organisms when administered by intraperitoneal (i.p.) injection and 5x10⁴ when administered orally in NaHCO₃. Mice were purchased from Charles River Breeding Laboratories, Inc. (Wilmington, Mass.) and were 5 to 6 weeks of age at initial challenge. All i.p. inoculations were performed as previously described, Miller et al., 1989, supra. Oral challenge experiments were performed with bacteria grown in LB broth and concentrated by centrifugation. The bacteria were resuspended in 0.1 M NaHCO₃ to neutralize stomach acid, and administered as a 0.5-ml bolus to animals under ether anesthesia. Colony counts were performed to accurately access the number of organisms administered. All challenge experiments were performed 1 month after i.p. inoculation and 6 weeks after oral challenge. Challenge inocula were administered by the same route as vaccinations. The care of all animals was under institutional guidelines as set by the animal are committees at the Massachusetts General Hospital and Harvard Medical School.

Protein electrophoresis was performed as follows. One-dimensional protein gel electrophoresis was performed by the method of Laemmli, 1970, Nature 227: 680, on whole-cell protein extracts of stationary-phase cells grown overnight in Luria broth. The gels were fixed and stained with Coomassie brilliant blue R250 in 10% acetic acid-10% methanol. Two-dimensional protein gel electrophoresis was performed by method of O'Farrell, 1975, J. Biol. Chem. 250:4007, on the same whole-cell extracts. Isoelectric focusing using 1.5% pH 3.5 to 10 ampholines (LKB Instruments, Baltimore, Md.) was carried out for 9,600 V h (700 V for 13 h 45 min). The final tube gel pH gradient extended from pH 4.1 to pH 8.1 as measured by a surface pH electrode (BioRad Laboratories, Richmond, calif.) and colored acetylated cytochrome pI markers (Calbiochem-Behring, La Jolla, Calif.) run in an adjacent tube. The slab gels were silver stained, merril et al., 1984, Methods Enzymol. 104:441.

In the macrophage survival assays experiments were performed as previously described, Miller et al., 1989, supra, by the method of Buchmeier et al., 1989, Infect. Immun. 57:1, as modified from the method of Lissner et al, 1983, J. Immunol. 131: 3006. Stationary-phase cells were opsonized for 30 min in normal mouse serum before exposure to the cultured bone marrow-derived macrophages harvested from BALB/c mice. One hour after infection, gentamicin sulfate (8 µg/ml) was added to kill extracellular bacteria. All time points were done in triplicate and repeated on three separate occasions.

### PhoP^{c} Mutant Strains Are More Effective as Live Vaccines

PhoP^{c} mutant *S. typhimurium* are very effective when used as a live vaccine against mouse typhoid fever and are superior to PhoP⁻ bacteria. As few a 15 PhoP^{c} bacteria protect mice against 10⁵ LD₅₀ (lethal doses 50%) of wild type organisms by the intraperitoneal route (Table 3). This suggests that *pag* gene products are important antigens for protective immunity against mouse typhoid. Preliminary results have documented that antigens recognized by serum of chronic typhoid carriers recognizes some *phoP*-regulated gene products of *S. typhi.* If protective antigens are only expressed within the host, then dead vaccines only grown in rich media may not induce an immune response against these proteins.

The use of different *S. typhimurium* dead vaccine preparations containing different mutations in the *phoP* regulon was evaluated. As can be seen in Table 3 no dead cell preparations (even those containing mixtures of PhoP- and PhoP^{c} bacteria) are as effective vaccines as are live bacteria. This suggests that there are other properties of live vaccines that increase immunogenicity or that important non-PhoP-regulated antigens are not in these preparations. The only protection observed in any animals studied was at the lowest challenge dose for those immunized with PhoP^{c} bacteria. This further suggests that *phoP* activated genes are important protective antigens.

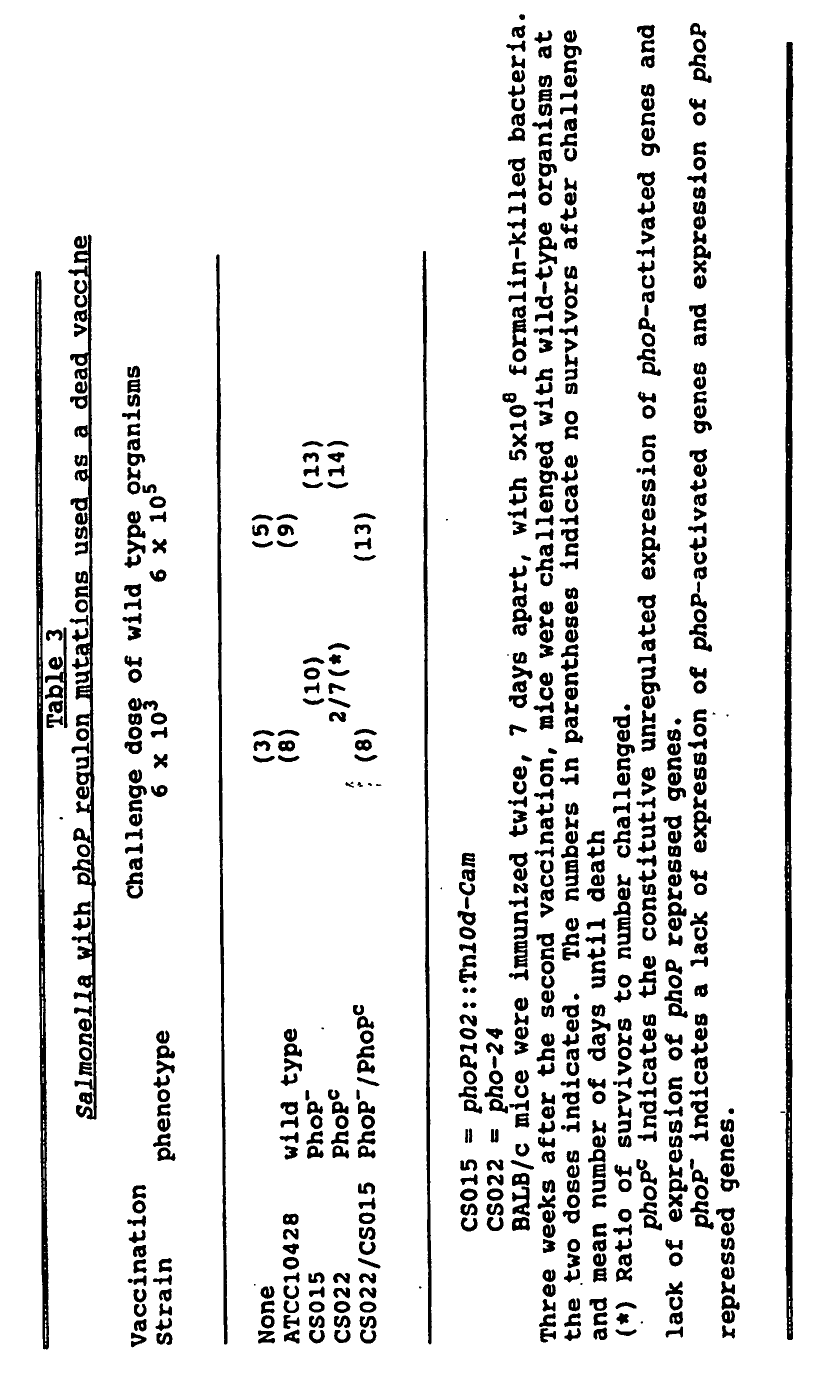

### aroA PhoP Regulon Double Mutant Strains

Recent efforts by Stocker, Levine, and colleagues have focused on the use of strains with auxotrophic mutations in aromatic amino acid and purine pathways as live vaccines, Hoseith et al., 1981, Nature 291:238, Stocker, 1988, Vaccine 6:141, hereby incorporated by reference, and Levine et al., 1987, J. Clin. Invest. 79: 888. Purine mutations were found to be too attenuating for immunogenicity, likely because purines are not available to the organism within the mammalian host, Sigwart et al., 1989, Infect. Immun. 57:1858. Because auxotrophic mutations may be complemented by homologous recombination events with wild type copies donated from environmental organisms or by acquiring the needed metabolite within the host, it would seem prudent for live vaccines to contain a second attenuating mutation in a different virulence mechanism, (i.e., not just a second mutation in the same metabolic pathway). Additionally, in mice the aroA mutants have some residual virulence. Various strains with aroA mutations combined with *phoP* regulon mutations were investigated for virulence attenuation and immunogenicity. Table 4 demonstrates that a PhoP⁻ or PhoP^{c} mutation further attenuates aroA mutant *S*. *typhimurium* by at least 100-fold and that, at least at high levels of vaccinating organisms, immunogenicity is retained. Strains with both a *pagC*⁻ and *phoP*^{c} phenotype are also further attenuated than either mutation alone. Therefore, *phoP* regulon mutations may increase the safety of *aro*A live vaccine preparations.

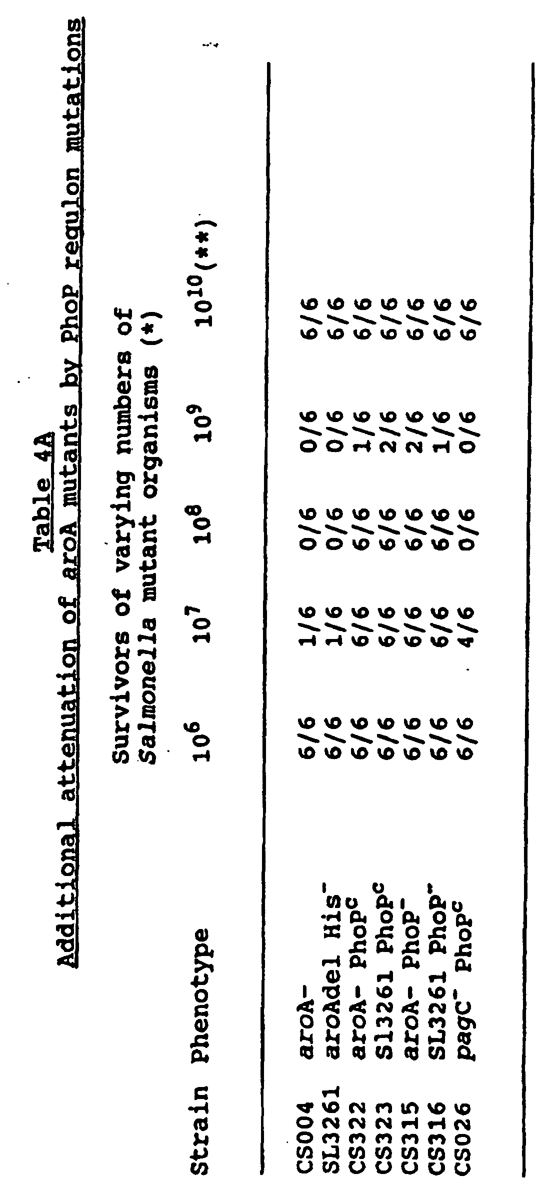

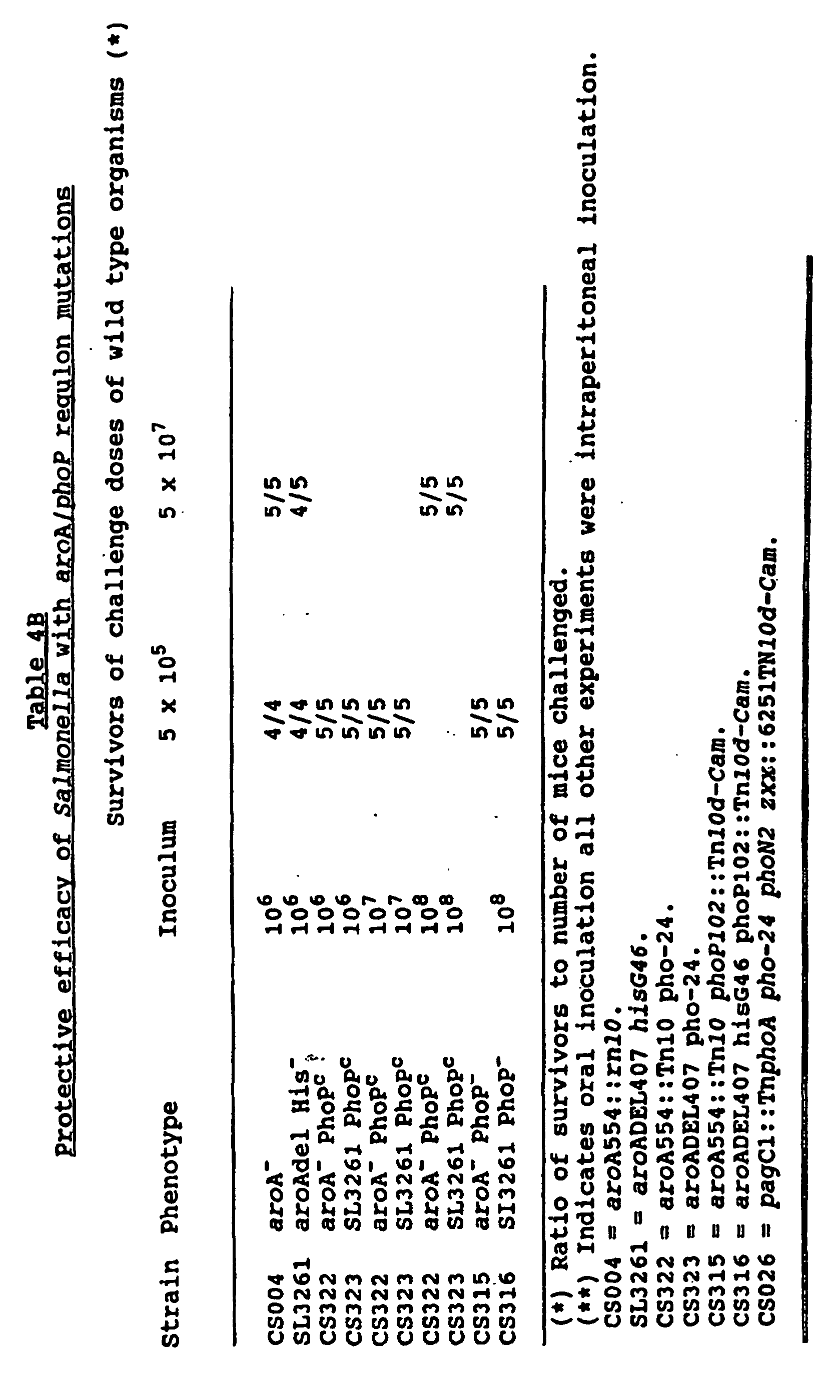

### Salmonella typhi phoP Regulon Mutations

The phoP regulon is at least partially conserved in *S. typhi* DNA hybridization studies as well as P22 bacteriophage transductional crosses have documented that the *phoP, phoQ*, and *pagC* genes appear highly conserved between *S. typhi* and *S*. *typhimurium* mutations in these genes in *S. typhi* have been made.

### Salmonella Live Vaccines as Delivery Systems for Heterologous Antigens

The vector used in the vaccine delivery system is a derivative of pJM703.1 described in Miller et al., 1988, J. Bact. 170:2575. This vector is an R6K derivative with a deletion in the *pir* gene. R6K derivatives require the protein product of the *pir* gene to replicate. *E*. *coli* that contain the *pir* gene present as a lambda bacteriophage prophage can support the replication of this vector. Cells that do not contain the *pir* gene will not support the replication of the vector as a plasmid. This vector also contains the mob region of RP4 which will allow mobilization into other gram negative bacteria by mating from *E. coli* strains such as SM101ambda *pir*, which can provide the mobilization function in trans.

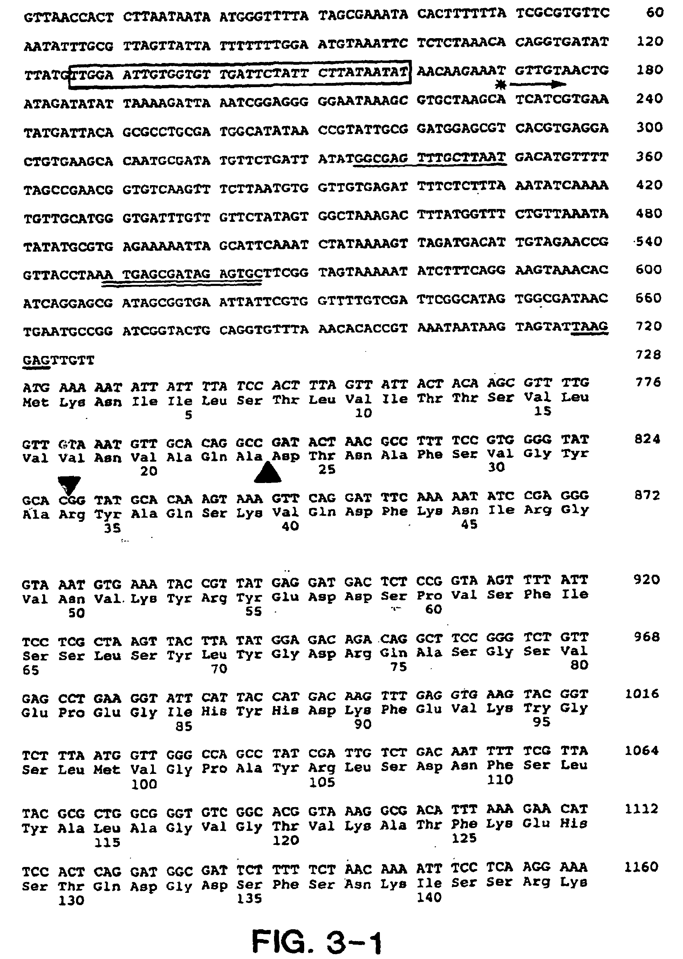

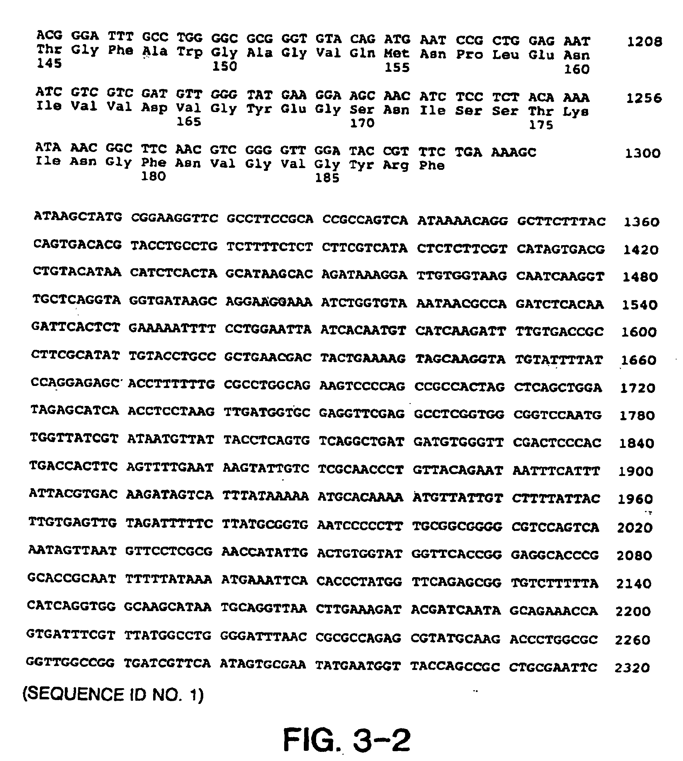

The *pag*C region is shown in Figs. 2 and 3. Fig. 2 shows the restriction endonuclease sites of the *pag*C locus. The heavy bar indicates *pag*C coding sequence. The TnphoA insertion is indicated by a inverted triangle. The direction of transcription is indicated by the arrow and is left to right. The numbers indicate the location of endonuclease sites, in number of base pairs, relative to the start codon of predicted *pag*C translation with positive numbers indicating location downstream of the start codon and negative numbers indicating location upstream of the start codon. A is AccI, B is *Bgl*I, C is *Cla*I, D is *Dra*I, *E* is *Eco*RI, H is *Hpa*I, N is *Nru*I, P is *Pst*I, *S* is *Ssp*I, T is *Stu*I, U is *Pvu*II, V is *Eco*RV, and II is *Bgl*II. Fig. 3 shows the DNA sequence (Sequence I.D. No. 1) and translation of *pag*C::Tn*phoA*. The heavy underlined sequence indicates a potential ribosomal binding site. The single and double light underlines indicate sequences in which primers were constructed complementary to these nucleotides for primer extension of RNA analysis. The asterisk indicates the approximate start of transcription. The arrow indicates the direction of transcription. The boxed sequences indicate a region that may function in polymerase binding and recognition. The inverted triangle is the site of the sequenced TnphoA insertion junction. The arrow indicates a potential site for single sequence cleavage.

3 kilobases of DNA containing the *pagC* gene (from the *PstI* restriction endonuclease site 1500 nucleotides 5' to the start of *pagC* translation to the *Eco*RI restriction endonuclease site 1585 nucleotides downstream of *pagC* translation termination) were inserted into the pJM703.1 derivative discussed above. The *pagC* sequence from the *Cla*I restriction endonuclease site was deleted (490 nucleotides) and replaced with a synthetic oligonucleotide polylinker that creates unique restriction endonuclease sites. DNA encoding one or more heterologous proteins, e.g., an antigen, can be inserted into this site. This creates a vector which allows the insertion of multiple foreign genes into the DNA surrounding *pag*C.

The vector can be mobilized into *Salmonella* by mating or any other delivery system, e.g., heat shock, bacteriophage transduction or electroporation. Since it can not replicate, the vector can only insert into *Salmonella* by site specific recombination with the homologous DNA on both sides of the *pagC* gene. This will disrupt and inactivate the native *pagC* locus and replace it with the disrupted *pagC* DNA carried on the vector.

Such recombination events can be identified by marker exchange and selective media if the foreign DNA inserted into the *pagC* locus confers a growth advantage. The insertion of antibiotic resistance genes for selection is less desirable as this could allow an increase in antibiotic resistance in the natural population of bacteria. Genes which confer resistance to substances other than antibiotics e.g., to heavy metals or arsenic (for mercury resistance, see Nucifora et al., 1989, J. Bact., 171 : 4241-4247), can be used to identify transformants. Alternatively, selection can be performed using a *Salmonella* recipient strain that carries an auxotrophic mutation in a metabolic pathway and a vector that carries DNA that complements the auxotrophic mutation. Many *Salmonella* live vaccine prototypes contain mutations in histidine or purine pathways thus complementation of these metabolic auxotrophies can be used to select for integrants. (Purine mutations specifically have been shown to be too attenuated for use in man.) Further proof of marker exchange can be documented by loss of the ampicillin resistance (carried on the plasmid backbone) or by blot hybridization analysis.

A gene useful for selection can be cloned by complementation of a vaccine strain with a metabolic auxotrophy. Specific examples include the cloning of the DNA encoding both *purB* and *phoP* by complementation of a strain deleted for function of both these genes. *Salmonella* gene libraries have been constructed in a pLAFR cosmid vector (Frindberg et al., 1984, Anal. Biochem. 137:266-267) by methods known to those skilled in the art. pLAFR cosmids are broad host range plasmids which can be mobilized into *Salmonella* from *E. coli.* An entire bank of such strains can be mobilized into *Salmonella* vaccine strains and selected for complementation of an auxotrophic defect (e.g., in the case of *purB* growth on media without adenine). The DNA able to complement this defect is then identified and can be cloned into the antigen delivery vector.

As discussed above heterologous genes can be inserted into the polylinker that is inserted into the *pag*C sequence of the vector. The heterologous genes can be under the control of any of numerous environmentally regulated promotor systems which can be expressed in the host and shut off in the laboratory. Because the expression of foreign proteins, especially membrane proteins (as are most important antigens), is frequently toxic to the bacterium, the use of environmentally regulated promoters that would be expressed in mammalian tissues at high levels but which could be grown in the laboratory without expression of heterologous antigens would be very desirable. Additionally, high expression of antigens in host tissues may result in increased attenuation of the organism by diverting the metabolic fuel of the organism to the synthesis of heterologous proteins. If foreign antigens are specifically expressed in host phagocytic cells this may increase the immune response to these proteins as these are the cells responsible for processing antigens.

The promoter systems likely to be useful include those nutritionally regulated promoter systems for which it has been demonstrated that a specific nutrient is not available to bacteria in mammalian hosts. Purines, Sigwart et al., 1989, Infect. Immun., 57:1858 and iron, Finklestein et al., 1983, Rev. Infect. Dis. 5:S759, e.g., are not available within the host. Promoters that are iron regulated, such as the aerobactin gene promoter, as well as promoters for biosynthetic genes in purine pathways, are thus excellent candidates for testing as promoters that can be shut down by growth in high concentrations of these nutrients. Other useful environmentally regulated *Salmonella* promoters include promoters for genes which encode proteins which are specifically expressed within macrophages, e.g., the DnaK and GroEL proteins, which are increased by growth at high temperature, as well as some *phoP* activated gene products, Buchmeier et al., 1990, Science 248:730. Therefore, promoters such as the *pagC* 5' controlling sequences and the better characterized promoters for heat shock genes, e.g., GroEL and DnaK, will be expected to be activated specifically within the macrophage. The macrophage is the site of antigen processing and the expression of heat shock genes in macrophages and the wide conservation of heat shock genes in nature may explain the immunodominance of these proteins. A consensus heat shock promoter sequence is known and can be used in the vectors (Cowling et al., 1985, Proc. Natl. Acad. Sci. USA 82:2679.

The vectors can include an environmentally regulated T7 polymerase amplification system to express heterologous proteins. For example, the T7 polymerase gene (cloned by Stan Tabor and Charles Richardson, See Current Protocols in Molecular Biology ed. Ausubel et al., 1989, (page 3.5.1.2) John Wiley and Sons) under control of an iron regulated promoter, can be included on the vectors described above. We have inserted the aerobactin gene promoter of *E*. *coli* with the sequence CATTTCTCATTGATAATGAGAATCATTATTGACATAATTGTTATTATTTTACC (SEQ ID NO:2), Delorenzo et al. J. Bact. 169:2624, in front of the T7 polymerase gene and demonstrated iron regulation of the gene product. This version of the vector will also include one or more heterologous antigens under the control of T7 polymerase promoters. It is well known that RNA can be synthesized from synthetic oligonucleotide T7 promoters and purified T7 in vitro. When the organism encounters low iron T7 polymerase will be synthesized and high expression of genes with T7 promoters will be facilitated.

### pagC-fusion proteins in S. typhimurium

Expression of heterologous antigens within macrophages under the control of *phoP* regulated promotors can be used as an effective method of both attenuating *Salmonellae* and enhancing immunogenicity of foreign antigens. As discussed above, the expression of PagC is induced in antigen processing cell, i.e., a macrophage. Thus, expression of a heterologous antigen under the control of the *pagC* promoter is also likely to be inducible in macrophages.

To evaluate the immune respone to a heterologous antigen expressed under the control of inducible *pag* promoters, mice were inoculated with bacteria which expressed the antigen, AP, under the control of the *pagC* or *pagD* regulatory sequences. Pag-AP fusion proteins were produced in these bacteria from a single chromosomal copy of the gene encoding AP. The bacteria were generated utilizing two methods: TnphoA mutagenesis, and genetic engineering techniques using a suicide vector, both of which are described above.

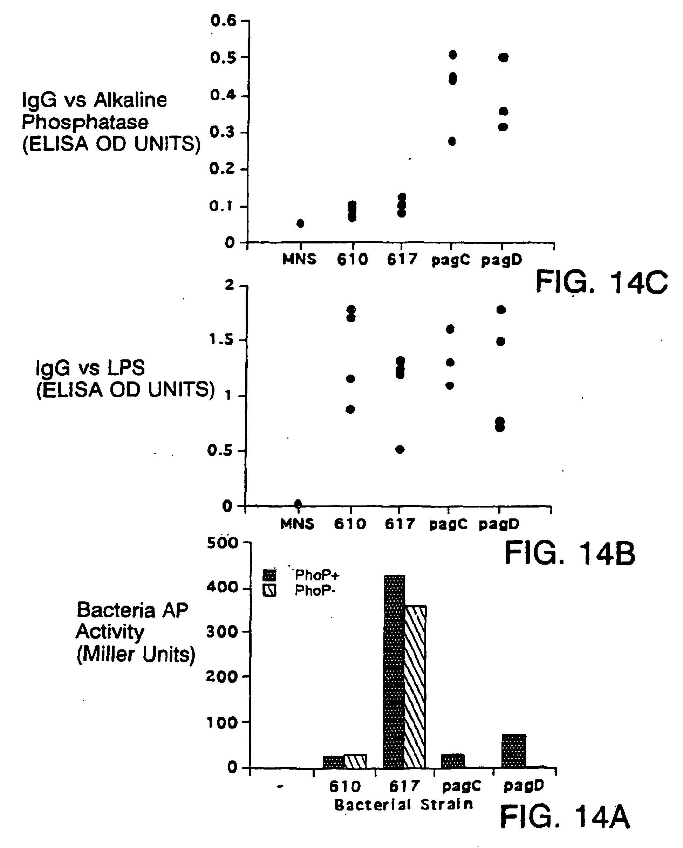

As a control, mice were innoculated with bacteria which expressed an AP fusion protein under the control of constitutive promoters. The constitutive promoter was completely independent of regulation by genes in the PhoP regulon. Two such strains of bacteria, Strain 610 and Strain 617, were constructed using methods described above. AP expression in Strain 610 was moderate, whereas AP expression in Strain 617 was high (see Fig. 14C).

These strains were injected intraperitoneally into BABL/C mice. Serum samples were taken three weeks after inoculation. Normal mouse serum (MNS) was used as a control. Standard ELISA assays were used to test the sera for the presence of AP-specific antibodies. Sera was also tested for LPS-specific antibodies using *S*. *typhimurium* LPS. Antibodies directed to LPS were detected in all the murine sera tested, but only those strains in which AP was expressed as a Pag fusion protein from a single chromosomal gene copy engendered an immune response against the model heterologous antigen, AP (see Figs. 14A and Fig. 148).

Despite approximately 10-fold higher constitutive expression of the AP fusion in strain 617, only a minimal immune response to this antigen was noted after immunization with strain 617. In contrast, a strong response was observed in mice inoculated with strains which expressed the Pag-AP fusion protein. These data indicate that *phoP*-regulation which results in *in vivo* induction of protein expression within macrophages increases the immunogenicity of heterologous antigens expressed under the control of the *pag* promoters. Any promoter which directs cell-specific, inducible expression of a protein in macrophages or other antigen presenting cells, e.g., *pag* described herein, can be used to increase the immunogencity of an antigen expressed in *Salmonella.*

### pagC/pagD promoter region; expression of heterologous proteins

*pagC* and *pagD* are divergently transcribed and are both PhoP activated. Other divergently transcribed, regulated genes are known in the art (Beck et al., 1988, Microbiol. Rev. 52:318-326), e.g., the *Klebsiella* pneumoniae *pulA-malX* region (Chapon et al., 1985, J. Bacteriol. 164:639-645). Transcription of most of such genes require accessory proteins, such as CAP, in addition to the regulator to activate transcription. These two genes are divergently transcribed, and their promoters are arranged back-to-back. A region of 134 bp exists between transcriptional start sites of these genes, which is similar to the intergenic region between *pagC* and *pagD.* The *pulA-malK* promoter region is predicted to contain two MalT (the regulatory protein of this system) binding sites, one for each gene. Other MalT-activated genes require the CAP protein for expression, but the *pulA* and *malX* genes do not, possibly because of the high local concentration of the MalT regulator. Since the region between the transcriptional start sites of *pagC* and *pagD* (the predicted -35 sequences) is only 137bp (nucleotides 562 to 776 of SEQ ID NO:15), it is likely that only PhoP binding sites exist in the intergenic region, and that binding of one or more phosphorylated PhoP molecules positively regulates both genes. This *pagC*/*pagD* intergenic region which contains the divergent promoters can be used to construct vectors to express two heterologous proteins, one in each direction.

### prg genes

As discussed above, *phoP*/*phoQ* constitutive mutations (phenotype PhoP^{c}) increase the expression of *pag* and repress the synthesis of approximately 20 proteins encoded by *phoP*-repressed genes (*prg*). PhoP^{c} bacteria are attenutated for mouse virulence suggesting that *prg* are virulence genes.

By use of the transposon, Tn*phoA*, five unlinked *prg* loci were identified. In general, media conditions (starvation) that activate *pag* expression repress *prg* expression. One *prg* locus, *prgH,* was demonstrated to contribute to mouse virulence by both the oral and the intraperitoneal route. Both PrgH as well as PhoP^{c} mutant *S. typhimurium* were found to be defective in induction of endocytosis by epithelial cells. Identification and mutation of such virulence genes will be useful in vaccine development.

### Nucleotide sequence of the prg H, prgI, prgJ, and prgK genes

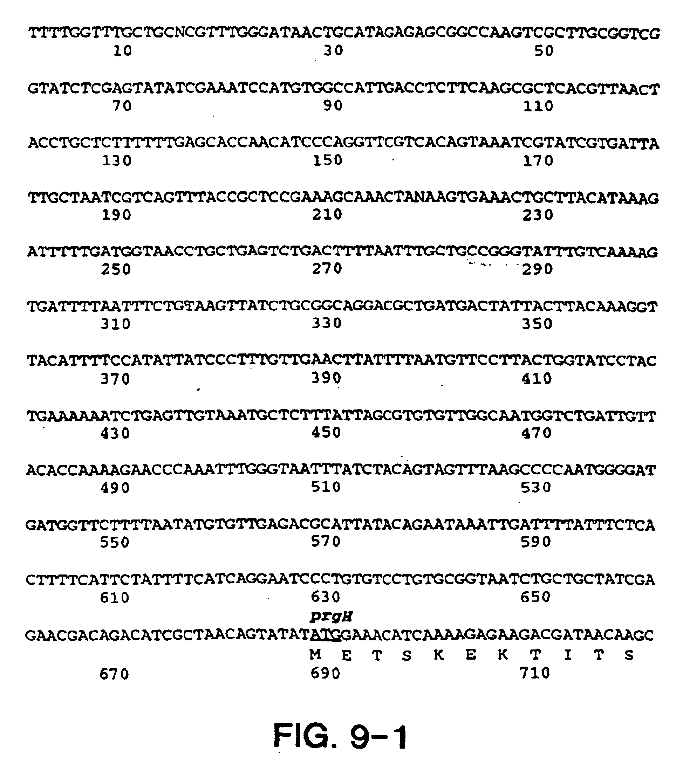

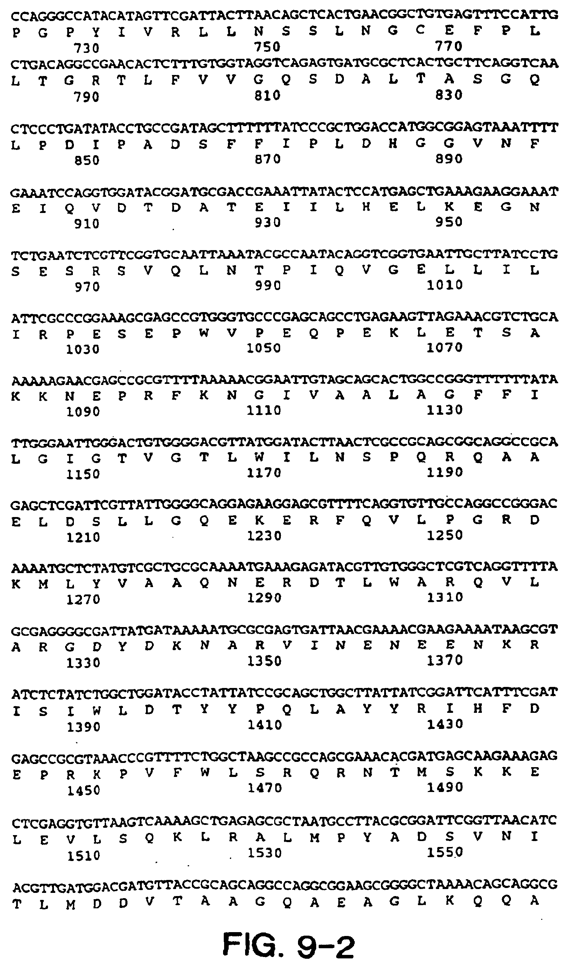

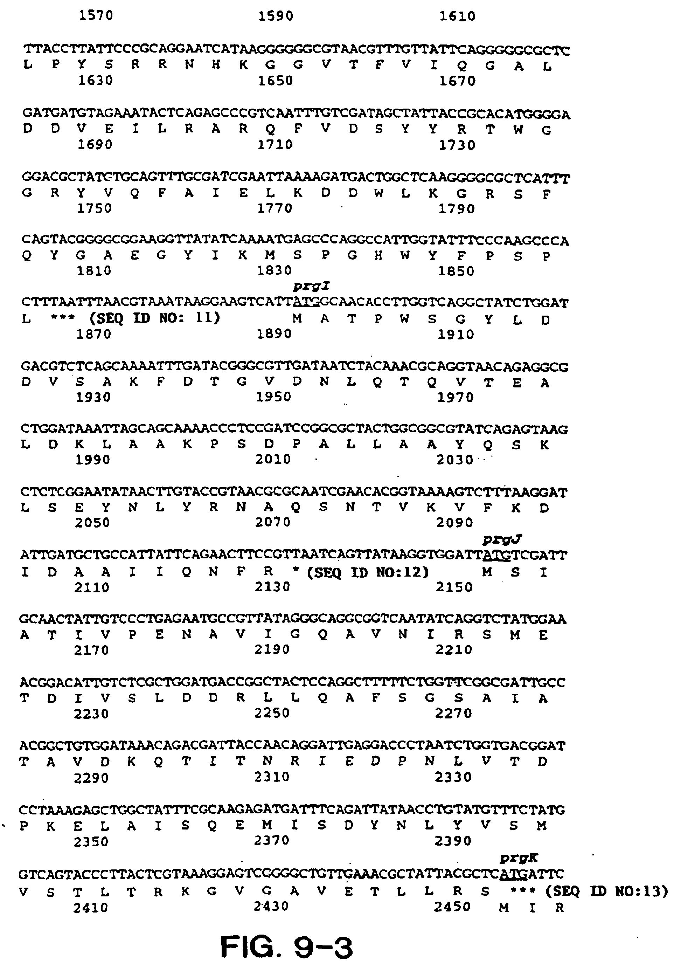

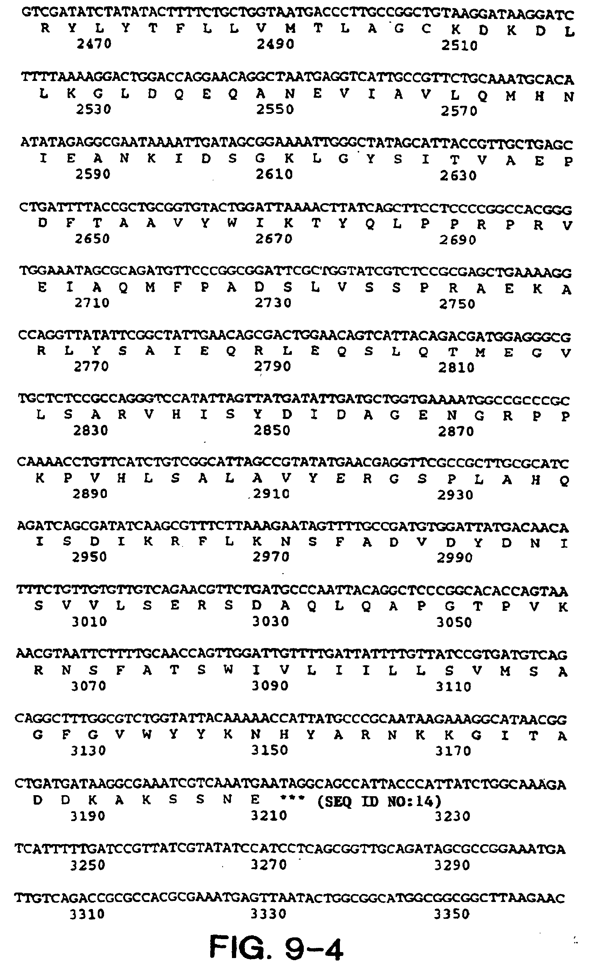

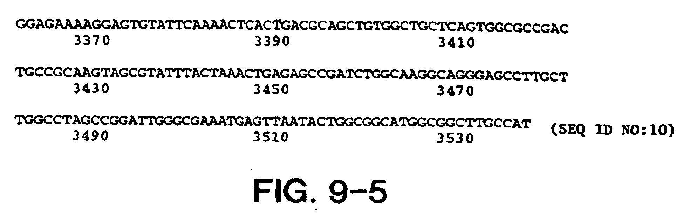

SEQ ID NO:10 represents the nucleotide sequence of a 5100-bp *Hind*III fragment that contains the hyperinvasive *hil* locus. Four ORFS encoding four *prg* genes are located within this DNA (see Fig. 9). The ATG start codon is underlined; the asteriks indicate the positions of the *prgH, prgI, prgJ,* and *prgk* stop codons. These *prg* loci are required for bacterial invasion of epithelial cells, full mouse virulence, and transepithelial neutrophil migration. A bacteria attenuated by a mutation in one or more of these loci can be used to vaccinate individuals against infection by the wild type pathogen.

### Strains, materials and methods

All bacterial strains used in the characterization of *prg* genes are listed in Table 5.

### Role of prg genes in virulence

The *prg* locus, *prgH*, was found to contribute to mouse virulence when *S*. *typhimurium* was administered by both the oral and intraperitoneal routes. PrgH as well as PhoP^{c} mutants were further found to be defective in bacterial-mediated uptake by epithelial cells suggesting that an inability to cross epithelial barriers might contribute to the attenuation of virulence observed. Competition studies in mice after oral ingestion of bacteria further supported that *prgH* mutants were defective in transcytosis across the intestinal epithelial barrier. Therefore, at least two phases of PhoP/PhoQ regulated protein expression essential to bacterial virulence have been defined. In one phase, *prg* expression promotes bacterial mediated endocytosis by epithelial cells (Table 10), while in another phase, *pag* expression promotes survival within macrophages.

Systemic pathogens, such as *Salmonella,* may encounter more complex and Varied environments than may be encountered by mucosal pathogens. The achievement of intermediate states of *pag* and *prg* expression could be essential to virulence at some stage of the infectious cycle. Consistent with this concept was the lack of uniformity observed in the expression of *pag* and *prg* on growth at different oxygen tensions and pH conditions. These data may also indicate that not all regulation of *pag* and *prg* is mediated directly through PhoP and PhoQ. Given the function of PhoP as a transcriptional regulator, it is likely that *prg* loci repression occurs at the level of transcription.

The approach of defining genes repressed by the *pho-24* mutation has led to the discovery of at least one virulence locus, *prgH*, which can be mutated to attentuate the bacteria for vaccine purposes.

### Attenuation of Bacterial Virulence by Constitutive Expression of Two-component Regulatory Systems

The virulence of a bacterium can be attenuated by inducing a mutation which results in the constitutive expression of genes under the control of a two-component regulatory system or by inducing a mutation that inactivates a gene under the control of the two-component systems. A balance between the expression of the genes under the control of the two-component system, e.g., between *pag* and *prg* gene expression, and possibly between two-component system regulated genes and other genes, is necessary for full virulence. Mutations that disrupt this balance, e.g., mutations that cause the constitutive expression of a gene under the control of the two-component system, or a mutation that inactivates a gene under the control of the two-component system, e.g., the *pag* gene, reduce virulence.

Constitutive mutations in two-component regulators can be identified by the use of a strain containing a recorder gene fusion to a gene regulated by the two-component system. Such gene fusions would most typically include DNA encoding the *lacZ* gene or AP fused to a gene under the control of the two-component system. Strains containing fusions that are (as compared to wild type or parental strains) highly expressed in an unregulated fashion, i.e., constitutive, can be detected by increased color on chromogenic substrates for the enzymes. To detect constitutive mutations a cloned virulence regulator could be mutagenized e.g., by passage through an *E. coli* strain defective in DNA repair or by chemical mutagenesis. The mutated DNA for the regulator would then be transferred to the strain containing the gene fusion and constitutive mutations identified by the high gene fusion expression (blue color in the case of a *lac*Z fusion grown on media containing X-gal). Constitutive mutations in a component of a two-component regulatory system could also be made by *in vitro* mutagenesis after other constitutive mutations have been sequenced and a specific amino acid change responsible for the constitutive phenotype identified. Putting several amino acid changes that all result in a PhoP constitutive phenotype would result in a decreased frequency of reversion by spontaneous base changes. A constitutive mutation could also be constructed by deletion of the portion of the amino terminus of the phospho-accepting regulator which contains the phosphoacceptor domain e.g., deletion of sequences encoding amino acids amino terminal to amino acid 119 in the *pho*P gene or deletion of analogous phospho accepting sequences in genes of other two-component regulatory systems. This could result in a conformational change similar to that induced by phosphorylation and result in increased DNA binding and transcriptional activation.

### Attenuation of virulence: deletion in the phoP/phoQ regulon

As discussed above, the PhoP regulon is essential to full virulence of *Salmonella.* This regulon is composed of two genes, PhoP and PhoQ located in an operon, and over 40 genes they positively and negatively regulate (pag and prg, respectively).

PhoP null *S. typhimurium* mutants have been demonstrated to be markedly attenuated and also effective vaccine strains when studied in the BALB/c mouse model of typhoid fever. This phenotype is likely the result of multiple, phoP-activated virulence genes, as transposon insertions in multiple different phoP-activated genes have been independently demonstrated to decrease *S*. *typhimurium* virulence. *S. typhimurium* mutants deleted for genes essential to aromatic amino acids (aroA null or aroC/aroD null mutants) are also markedly attenuated in the mouse model. However, testing of aroC/aroD mutants in humans has shown that although these strains are immunogenic, bacteremias and side effects such as fever have been noted at doses as low as 10⁵ to 10⁷ organisms administered as a single oral dose (J. Clin. Invest. 90:412-420).

It has now been found that a large deletion in a global regulator of *Salmonella* virulence, i.e., the PhoP/PhoQ operon, significantly decreases the virulence of the bacteria. This mutation, the result of a 1 kB deletion of DNA within the *phoP*/*phoQ* locus, was initially made in *S. typhimurium* and subsequently transferred via homologous recombination to *S. typhi.* In order to confer an even greater margin of safety in construction of this vaccine, it was created in a strain background deleted for genes essential to aromatic amino acids and carrying the histidine G46 mutation, a mutation rendering the organism auxotrophic for histidine. The resulting strain, *S. typhi* TyLK445, offers several advantages over existing vaccine candidates, most notably, immunogenicity without transient bactermia.

### Use

The *Salmonella* cells of the invention are useful as sources of immunological protection against diseases, e.g., typhoid fever and related diseases, in an animal, e.g., a mammal, e.g., a human, in particular as the basis of a live-cell vaccine capable of colonizing the inoculated animal's intestine and provoking a strong immune reaction. Appropriate dosages and conditions of administration of such a live, attenuated vaccine are known in the art, e.g., as described in Holem et al., Acute Enteric Infections in Children, New Prospects for Treatment and Prevention (1981) Elsevier/North-Holland biomedical Press, Ch. 26, pp. 443 et seq. (Levine et al.), and are described in the examples below.

### Advantages

One advantage of the invention is that the bacterial cells are attenuated as a result of a mutation(s), i.e., the *phoP*/*phoQ* operon, that directly affect a virulence pathway. Another advantage is that the bacterial cells have mutations in two completely different attenuating genes, i.e., the aromatic amino acid synthesis pathway (*Aro*), and in an operon important to *Salmonella* virulence (*PhoP*/*Q*). As a result, the bacteria appear to be extremely attenuated; doses as high as 1 × 10⁹ cfu appear to be very safe. Other vaccines under development, such as CVD 908, have caused some systemic symptoms, e.g., fever or bacteremia, at doses as low as 1 × 10⁷ cfu.

In addition to the *phoP*/*phoQ* deletion and the AroA-mutation, the bacterial cells of the invention may also contain a histidine mutation to further alternate virulence, although absence of the histidine mutation may improve immunogenicity. The bacterial cells of the invention are the most promising vaccine candidates to date because they are strongly immunogenic and safe, i.e., extremely attenuated.

### EXAMPLE 1: Construction of vaccine strain

The bacterial cells of the invention were made by deleting approximately 1 kb of DNA in the *phoP*/*phoQ* regulon.

*PhoP*/*phoQ* deleted suicide vectors were constructed using methods known in the art. A DNA fragment containing the *phoP*/*phoQ* locus was obtained by PCR using wild type *S*. *typhimurium* chromosomal DNA as a template. PCR primers flanking the *phoP*/*phoQ* locus were engineered to contain terminal restriction enzyme recognition sites, e.g., recognition site for EcoRI, to facilitate subsequent cloning. Following amplification, the PCR product was digested with EcoRI and cloned into the EcoRI site in the polylinker of a high copy vector. The plasmid containing the *phoP*/*phoQ* DNA fragment was named pLH356.

Sequence analysis and restriction mapping of the *phoP*/*phoQ* locus revealed four HpaI sites within the locus; no HpaI sites were found in the vector. To create an internal deletion within the *phoP*/*phoQ* locus, pLH356 DNA was cut to completion with HpaI, and religated, to yield with an internal deletion from nucleotides 376-1322 (pLH418). This deletion was confirmed by restriction digestion of the plasmid.

A DNA fragment containing the internally deleted *phoP*/*phoQ* locus was excised from pLH418 using the SacI/SphI restriction sites within the polylinker region of the vector. This fragment was cloned into compatible sites in the plasmid CVD442, which carries the *sacB* gene to allow positive selection for allelic exchange. The resulting suicide vector was called pLH423.

pLH423 was transformed into *E*. *coli* lambda *pir* SY327, and subsequently into *E. coli* lambda *pir* SM10 (strain LH425). *E. coli* strain LH425 was mated with *S*. *typhimurium* strain CS019. single recombinants carrying plasmid sequences integrated onto the *S*. *typhimurium* chromosome were selected by plating on agar containing ampicillin and chloramphenicol (Strain LH428). These strains were confirmed to be ampicillin resistant and sucrose sensitive, i.e., death on 20% sucrose plates containing no NaCl when incubated at 30°C. These data confirm the integration of plasmid sequences into the *Salmonella* chromosome.

A P22 bacteriophage lysate was made from strain LH428; phage particles were concentrated 20× by high speed centrifugation and transduced into *S*. *typhi* strain 522Ty2 (a strain with a deletion in the *aroA gene*, and the G646 mutation which renders the organism auxotrophic for histidine). Single recombinant *S*. *typhi* organisms were selected by plating on LB plates supplemented with aromatic amino acids, cystine, histidine, and ampicillin (strain LH435).

Strain LH453 was grown with aromatic amino acids, cystine, and histidine (but without ampicillin) to mid logarithmic growth phase. Serial dilutions were plated on LB 20% sucrose plates lacking NaCl, and on LB plates lacking NaCl. The number of bacteria that grew on plates without sucrose was greater than the number that grew on sucrose-supplemented plates by a factor of three logs. These data suggest that many colonies lost plasmid sequences containing the sacB gene.

Multiple colonies from the sucrose selection were picked and confirmed to be ampicillin sensitive and sucrose resistant. Chromosomal DNA from approximately 10 colonies was purified and subjected to Southern blot analysis, utilizing the 2.3 kb fragment of wild type *pboP*/*pboQ* as a probe.

Southern blotting revealed the loss of two HpaI sites and an XmnI site known to be within the 1 kb deleted fragment of *phoP*/*phoQ* in several strains. One of these strains was designated TyLH445.

### EXAMPLE 2: in vitro evaluation of TyLH445

TyLH445 was extensively characterized *in vitro* using standard clinical microbiological tests. The nutritional requirements of TyLH445 were evaluated. TyLH445 did not grow on M-9 plates unless supplemented with aromatic amino acid mix, cystine (*S. typhi* grows better with cystine), and histidine. These data confirmed that TyLH445 was AroA-, His-.

TyLH445 was found to agglutinate with polyclonal serum against *Salmonella* and polyclonal serum against *S*. *typhi Vi* antigen. Group D agglutination was found to be variable, perhaps due to excess Vi antigen. TyLH445 was also found to be indole negative (as are all *Salmonellae*), and to produce very little hydrogen sulfide (as do many *S. typhi*). Biochemical testing utilizing both the VITEK system as well as the BBL Crystal Enteric organism identification system was also carried out. These data indicated that the TyLH445 strain was *S*. *typhi.*

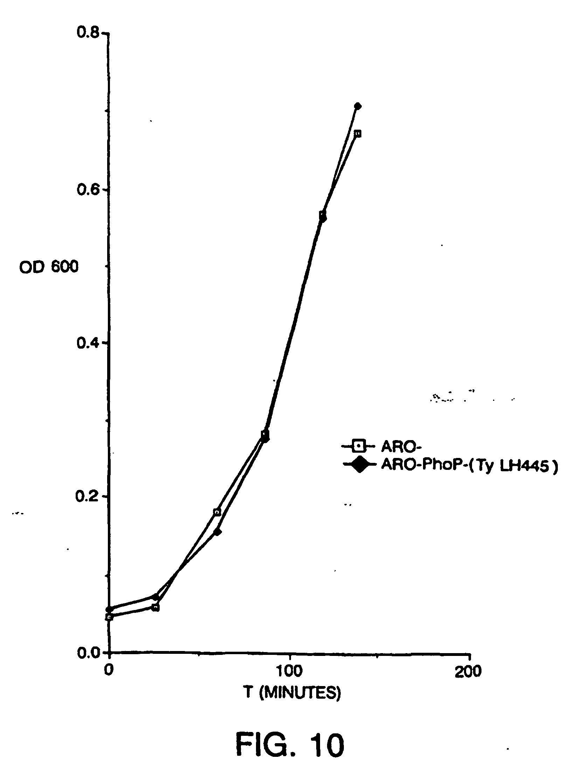

Growth characteristics of TyLH445 were also evaluated. TyLH445 was found to grow just as quickly as its parent, 522Ty2, (*phoP*/*phoQ* locus intact). Growth in vitro was measured in aromatic amino acid/histidine/cystine-supplemented Luria broth at 37°C. Growth curves of the parent and vaccine strain were found to be essentially identical (see Fig. 10).

Standardized clinical testing methods were use to determine antibiotic sensitivity. TyLH445 and the parent strain, 522Ty2, were found to be sensitive to ampicillin, trimethoprim-sulfamethoxazole, ciprofloxacin, aminoglycosides, and third generation cephalosporins. No difference in zone sizes was detected between the parent and vaccine strains, suggesting that no other antibiotic resistance mechanisms, e.g., modification of antibiotic transport systems, or modification of the cell wall of the bacterium, were affected by introduction of the mutated *phoP*/*phoQ* locus into *S*. *typhi.*

The *phoP*/*phoQ* HpaI deletion mutants were tested for defensin sensitivity, a phenotype of *PhoP* null mutants. Defensin sensitivity assays were performed as follows.

Liquid cultures of strains to be tested were grown overnight. Cultures were then diluted 1:200, and grown to an optical density (OD₆₀₀) of approximately 0.2, after which the cells were diluted to concentration of approximately 1 x 10⁵ organisms per 0.05 ml.

Two reactions were carried out for each strain: (1) vehicle alone (0.01% acetic acid in sterile water) and (2) defensin NP-1 solution (70 ug/ml in 0.01% acetic acid). An equal volume of bacterial suspension in tryptone was added and the test tubes were incubated on a roller at 37°C for 2 hours. The final volume in each reaction tube was 0.1 ml, making the final concentration of defensin 35 ug/ml.

Defensin is inactivated by the high salt and high protein concentration present in bacterial growth media, e.g. LB broth. Thus, defensin activity was stopped by adding 900 ul of Luria broth to each tube. Serial dilutions of each tube were plated and cfu/ml was determined for both the control tube and treatment tube for each strain. Results were expressed as log of bacteria killed for each strain. Typically, 1.0-1.5 log of wild type bacteria were killed. *PhoP* null mutants generally exhibit 2-4 logs of killing. Since strains with slower growth rates appear less susceptible to defensin killing, the growth rate of each strain tested in the defensin sensitivity assay was measured. Strains with similar growth rates were compared in the defensin sensitivity assay.

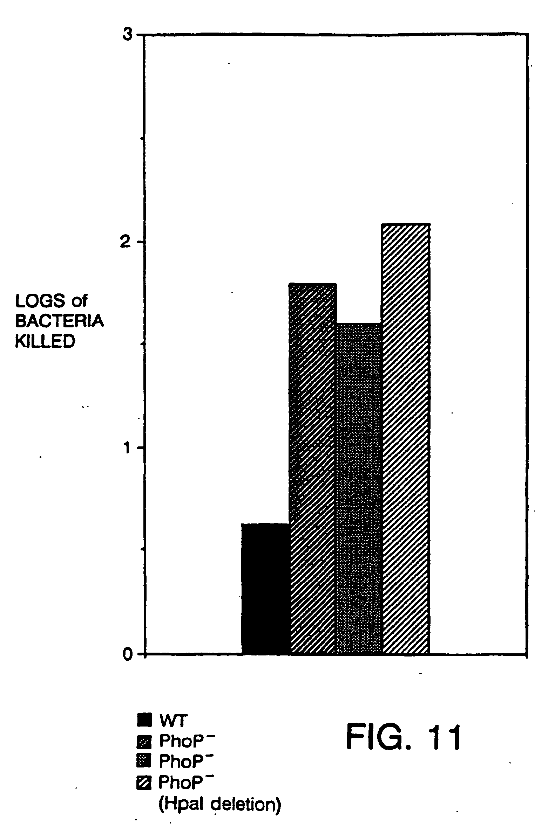

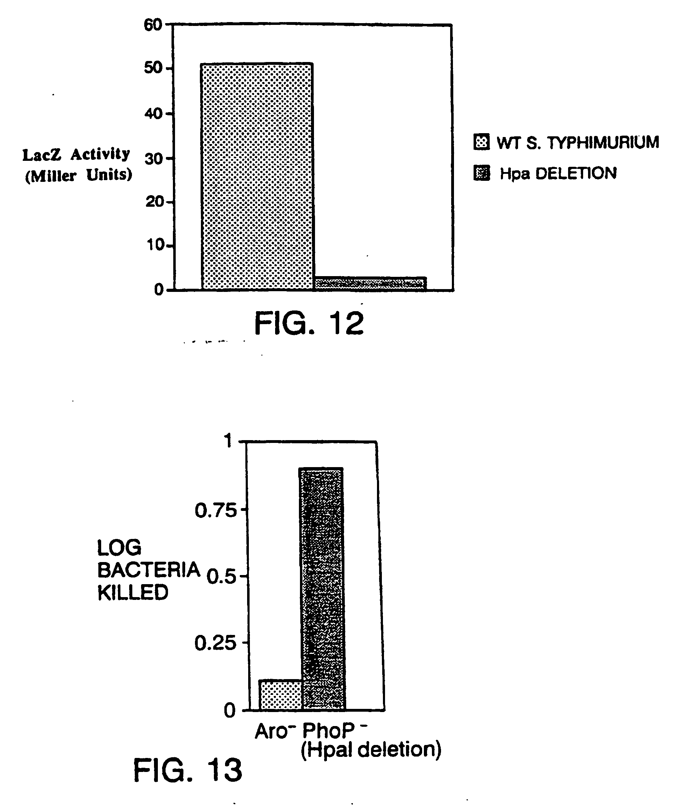

The HpaI deletion was evaluated both in an *S*. *typhimurium* background and in the *S. typhi* background. In both backgrounds, the deletion mutation conferred sensitivity to rabbit defensin NP-1 at a concentration of 35 ug/ml. See Fig. 11 and Fig. 13. The difference between *PhoP*+ and HpaI deleted *PhoP* null mutants was less pronounced in the *S. typhi* strain, an effect that may reflect the slower growth rate of the less hardy *S. typhi* strain compared to the *S. typhimurium* strain which lacks the additional auxotrophies.

The state of *phoP* activation in bacteria with the HpaI *phoP*/*phoQ* deletion was tested utilizing a *LacZ* recorder gene fused to *phoP*-activated gene B (*pagB*). Since the efficiency of transduction utilizing P22 in *S*. *typhi* is low, these studies were performed in *S*. *typhimurium* rather than *S*. *typhi. PhoP* activation was found to be 40-60 Miller units (Miller et al., 1972, Experiments in Molecular Genetics, Cold Spring Harbor Laboratory, Cold Spring Harbor, N.Y., pp. 352-355) in the presence of an intact *phoP*/*phoQ* locus, and just barely detectable in strains with the HpaI deletion (3cfusee Fig. 12).

### EXAMPLE 3: In vivo evaluation of S. typhimurium HpaI deleted strain

As *S. typhi* strains are not pathogenic for mice, the HpaI *phoP*/*phoQ* deletion mutation was evaluated in both wild type and *aroA- S. typhimurium.* Female BALB/c mice were injected intraperitoneally with various dilutions of *S*. *typhimurium* LH430, a wild type *S*. *typhimurium* carrying the HpaI deletion. The LD₅₀ of this strain was determined to be between 8.2 x 10⁵ and 8.2 x 10⁶. (All mice receiving 8.2 x 10⁵ cfu survived, and all receiving 8.2 x 10⁶ died.) These data are consistent with the LD₅₀ data obtained with strains harboring transposon insertions at the *phoP*/*phoQ* locus.

Immunogenicity of the HpaI *phoP*/*phoQ* deletion was evaluated in *S*. *typhimurium aro*A*::tet* (LH481), a strain comparable to the human vaccine strain. Mice were inoculated intraperitoneally with 2.3 x 10⁵ and 2.3 x 10⁶ cfu of LH481 (4 mice per vaccine dose), and challenged 30 days later with 30 × the LD₅₀ of wild type organisms. All mice but one mouse survived. The mouse that died was in the group that received the lower vaccine dose. No animal receiving the higher vaccine dose became ill.

### EXAMPLE 4: Phase I study human studies

The vaccine strain was administered to human volunteers at doses of 1 × 10⁵ to 1 × 10¹⁰ cfu/single oral dose. Two volunteers received each dose; 3 volunteers were given a dose of 1 × 10⁸ cfu/ml. Volunteers were evaluated at various time points following administration of the vaccine.

### Safety

To detect the presence of the vaccine strain in patient blood, Bactec blood cultures were performed in duplicate on days 4, 6, 8, 10, 12 after taking vaccine. Bacteremia was not detected in any of the volunteers.

Thirteen adult human volunteers have received escalating single oral doses of this new attenuated typhoid fever vaccine. No individuals have had side effects of any sort. Specifically, there have been no fevers, no gastrointestinal symptoms, and no constitutional symptoms. Volunteers have been subjected to serial blood cultures on a preset schedule after receiving the oral vaccine 2 sets of BACTEC blood cultures performed on each of days 4, 6, 8, 10 and 12 after receiving the vaccine, and no positive blood cultures have been noted. Volunteers have been followed up at 2 months after receiving the vaccine, and no late symptoms have been reported.

### Colonization

Stool samples were tested for the presence of the vaccine strain TyLH455 using methods known in the art. Primary stool was evaluated for the presence of the vaccine strain on culture plates. In some cases, it was necessary to enrich stool samples for the vaccine strain by incubating the stool overnight in BBL Selenite F broth supplemented with Aro/His/cystine in order to detect the bacteria. This medium is somewhat inhibitory for *E*. *coli* and but promotes *Salmonella* growth.

Volunteers have been colonized for various time periods from 1-6 days after receiving the vaccine. With the highest doses (10⁹ or 10¹⁰) volunteers have had positive primary culture plates in the initial 1-3 days post vaccination, whereas at lower doses, only selenite enrichment broth cultures (selective medium for *Salmonella* which inhibits other enterics) have been positive for the vaccine organism. No volunteer studied thus far has had prolonged carriage of the vaccine organism at 2 months of followup.

**Table 17**

| **Dose** | **Number** | **Colonization** |
|---|---|---|
| 10⁵ | 2 | NO |
| 10⁶ | 2 | 2/2 for 1-2 days |
| 10⁷ | 2 | 1/2 for 3 days |
| 10⁸ | 3 | 1/3 for 6 days |
| 10⁹ | 2 | 2/2 for 4-6 days both had positive primary plates day 1 |
| 10^{10**} | 2 | 2/2 for 3-6 days both had positive primary plates on days 1 and 2 |

| | | |
|---|---|---|
| * Measured by whole cell and LPS ELISAs and Widal test vs. H flagellar antigen. Sera analyzed at 1:40 and higher dilutions in all tests. | | |
| ** One of these volunteers has received a booster dose of 10¹⁰ organisms, given one month after the primary inoculation (serologies pending). | | |

### Immunogenicity

Induction of an immune response to the vaccine strain was measured by standard ELISA assays. Sera was collected from volunteers 0, 7, 14, 21, and 28 days after receiving a single oral dose of the vaccine. ELISA assays were carried out using whole bacteria TyLH445 and *S. typhi* LPS (SIGMA, St. Louis, MO) as antigens. Day 0 serum from each volunteer was used as an internal negative control. Convalescent sera from patients previously infected with wild type *S*. *typhi* (most from Mexico) were used as positive controls.

Several volunteers had documented seroconversion at 21 days after receiving the vaccine, as measured by ELISA in which IgG antibodies directed against whole vaccine organisms or against *S. typhi* LPS were detected. Sera taken from patients prior to administration of the vaccine (pre-immune sera) were tested, and the data used to establish a baseline. Patient sera taken at various time points after vaccination were considered positive if the test results were 0.2 ELISA OD units greater than that of the preimmune serum.

### SEQUENCE LISTING

**(1) GENERAL INFORMATION:**
   **(i) APPLICANT:** Miller, Samuel I.
      Mekalanos, John J.
   **(ii) TITLE OF INVENTION:** SALMONELLA VACCINES
   **(iii) NUMBER OF SEQUENCES:** 15
   **(iv) CORRESPONDENCE ADDRESS:**
      **(A) ADDRESSEE:** Fish & Richardson
      **(B) STREET:** 225 Franklin Street
      **(C) CITY:** Boston
      **(D) STATE:** Massachusetts
      **(E) COUNTRY:** U.S.A.
      **(F) ZIP:** 02110-2804
   **(v) COMPUTER READABLE FORM:**
      **(A) MEDIUM TYPE:** Floppy disk
      **(B) COMPUTER:** IBM PC compatible
      **(C) OPERATING SYSTEM:** PC-DOS/MS-DOS
      **(D) SOFTWARE:** PatentIn Release #1.0, Version #1.25 and WordPerfect (Version 5.1)
   **(vi) CURRENT APPLICATION DATA:**
      **(A) APPLICATION NUMBER:**
      **(B) FILING DATE:**
      **(C) CLASSIFICATION:**
   **(vii) PRIOR APPLICATION DATA:**
      **(A) APPLICATION NUMBER:** 08/090,526
      **(B) FILING DATE:** July 9, 1993
   **(viii) ATTORNEY/AGENT INFORMATION:**
      **(A) NAME:** Clark, Paul T.
      **(B) REGISTRATION NUMBER:** 30,162
      **(C) REFERENCE/DOCKET NUMBER:** 00786/220001
   **(ix) TELECOMMUNICATION INFORMATION:**
      **(A) TELEPHONE:** (617) 542-5070
      **(B) TELEFAX:** (617) 542-8906
      **(C) TELEX:** 200154
**(2) INFORMATION FOR SEQUENCE IDENTIFICATION NUMBER:** 1:
   **(i) SEQUENCE CHARACTERISTICS:**
      **(A) LENGTH:** 2320
      **(B) TYPE:** nucleic acid
      **(C) STRANDEDNESS:** double
      **(D) TOPOLOGY:** linear
   **(ii) MOLECULE TYPE:** DNA (genomic)
   **(xi) SEQUENCE DESCRIPTION:** SEQ ID NO: 1: 
GTTAACCACT CTTAATAATA ATGGGTTTTA TAGCGAAATA CACTTTTTTA TCGCGTGTTC 60

AATATTTGCG TTAGTTATTA TTTTTTTGGA ATGTAAATTC TCTCTAAACA CAGGTGATAT 120

TTATGTTGGA ATTGTGGTGT TGATTCTATT CTTATAATAT AACAAGAAAT GTTGTAACTG 180

ATAGATATAT TAAAAGATTA AATCGGAGGG GGAATAAAGC GTGCTAAGCA TCATCGTGAA 240

TATGATTACA GCGCCTGCGA TGGCATATAA CCGTATTGCG GATGGAGCGT CACGTGAGGA 300

CTGTGAAGCA CAATGCGATA TGTTCTGATT ATATGGCGAG TTTGCTTAAT GACATGTTTT 360

TAGCCGAACG GTGTCAAGTT TCTTAATGTG GTTGTGAGAT TTTCTCTTTA AATATCAAAA 420

TGTTGCATGG GTGATTTGTT GTTCTATAGT GGCTAAAGAC TTTATGGTTT CTGTTAAATA 480

TATATGCGTG AGAAAAATTA GCATTCAAAT CTATAAAAGT TAGATGACAT TGTAGAACCG 540

GTTACCTAAA TGAGCGATAG AGTGCTTCGG TAGTAAAAAT ATCTTTCAGG AAGTAAACAC 600

ATCAGGAGCG ATAGCGGTGA ATTATTCGTG GTTTTGTCGA TTCGGCATAG TGGCGATAAC 660

TGAATGCCGG ATCGGTACTG CAGGTGTTTA AACACACCGT AAATAATAAG TAGTATTAAG 720

GAGTTGTT 728

ATG AAA AAT ATT ATT TTA TCC ACT TTA GTT ATT ACT ACA AGC GTT TTG 776
Met Lys Asn Ile Ile Leu Ser Thr Leu Val Ile Thr Thr Ser Val Leu
                5                   10                  15

GTT GTA AAT GTT GCA CAG GCC GAT ACT AAC GCC TTT TCC GTG GGG TAT 824
Val Val Asn Val Ala Gln Ala Asp Thr Asn Ala Phe Ser Val Gly Tyr
            20                  25                  30

GCA CGG TAT GCA CAA AGT AAA GTT CAG GAT TTC AAA AAT ATC CGA GGG 872
Ala Arg Tyr Ala Gln Ser Lys Val Gln Asp Phe Lys Asn Ile Arg Gly
        35                  40                  45

GTA AAT GTG AAA TAC CGT TAT GAG GAT GAC TCT CCG GTA AGT TTT ATT 920
Val Asn Val Lys Tyr Arg Tyr Glu Asp Asp Ser Pro Val Ser Phe Ile
    50                  55                  60

TCC TCG CTA AGT TAC TTA TAT GGA GAC AGA CAG GCT TCC GGG TCT GTT 968
Ser Ser Leu Ser Tyr Leu Tyr Gly Asp Arg Gln Ala Ser Gly Ser Val
65                  70                  75                  80
 
GAG CCT GAA GGT ATT CAT TAC CAT GAC AAG TTT GAG GTG AAG TAC GGT 1016
Glu Pro Glu Gly Ile His Tyr His Asp Lys Phe Glu Val Lys Try Gly
85 90 95

TCT TTA ATG GTT GGG CCA GCC TAT CGA TTG TCT GAC AAT TTT TCG TTA 1064
Ser Leu Met Val Gly Pro Ala Tyr Arg Leu Ser Asp Asn Phe Ser Leu
100 105 110

TAC GCG CTG GCG GGT GTC GGC ACG GTA AAG GCG ACA TTT AAA GAA CAT 1112
Tyr Ala Leu Ala Gly Val Gly Thr Val Lys Ala Thr Phe Lys Glu His
115 120 125

TCC ACT CAG GAT GGC GAT TCT TTT TCT AAC AAA ATT TCC TCA AGG AAA 1160
Ser Thr Gln Asp Gly Asp Ser Phe Ser Asn Lys Ile Ser Ser Arg Lys
130 135 140

ACG GGA TTT GCC TGG GGC GCG GGT GTA CAG ATG AAT CCG CTG GAG AAT 1208
Thr Gly Phe Ala Trp Gly Ala Gly Val Gln Met Asn Pro Leu Glu Asn
145 150 155 160

ATC GTC GTC GAT GTT GGG TAT GAA GGA AGC AAC ATC TCC TCT ACA AAA 1256
Ile Val Val Asp Val Gly Tyr Glu Gly Ser Asn Ile Ser Ser Thr Lys
165 170 175

ATA AAC GGC TTC AAC GTC GGG GTT GGA TAC CGT TTC TGA AAAGC 1300
Ile Asn Gly Phe Asn Val Gly Val Gly Tyr Arg Phe
180 185

ATAAGCTATG CGGAAGGTTC GCCTTCCGCA CCGCCAGTCA ATAAAACAGG GCTTCTTTAC 1360

CAGTGACACG TACCTGCCTG TCTTTTCTCT CTTCGTCATA CTCTCTTCGT CATAGTGACG 1420

CTGTACATAA CATCTCACTA GCATAAGCAC AGATAAAGGA TTGTGGTAAG CAATCAAGGT 1480

TGCTCAGGTA GGTGATAAGC AGGAAGGAAA ATCTGGTGTA AATAACGCCA GATCTCACAA 1540

GATTCACTCT GAAAAATTTT CCTGGAATTA ATCACAATGT CATCAAGATT TTGTGACCGC 1600

CTTCGCATAT TGTACCTGCC GCTGAACGAC TACTGAAAAG TAGCAAGGTA TGTATTTTAT 1660

CCAGGAGAGC ACCTTTTTTG CGCCTGGCAG AAGTCCCCAG CCGCCACTAG CTCAGCTGGA 1720

TAGAGCATCA ACCTCCTAAG TTGATGGTGC GAGGTTCGAG GCCTCGGTGG CGGTCCAATG 1780

TGGTTATCGT ATAATGTTAT TACCTCAGTG TCAGGCTGAT GATGTGGGTT CGACTCCCAC 1840

TGACCACTTC AGTTTTGAAT AAGTATTGTC TCGCAACCCT GTTACAGAAT AATTTCATTT 1900

ATTACGTGAC AAGATAGTCA TTTATAAAAA ATGCACAAAA ATGTTATTGT CTTTTATTAC 1960

TTGTGAGTTG TAGATTTTTC TTATGCGGTG AATCCCCCTT TGCGGCGGGG CGTCCAGTCA 2020

AATAGTTAAT GTTCCTCGCG AACCATATTG ACTGTGGTAT GGTTCACCGG GAGGCACCCG 2080

GCACCGCAAT TTTTTATAAA ATGAAATTCA CACCCTATGG TTCAGAGCGG TGTCTTTTTA 2140

CATCAGGTGG GCAAGCATAA TGCAGGTTAA CTTGAAAGAT ACGATCAATA GCAGAAACCA 2200

GTGATTTCGT TTATGGCCTG GGGATTTAAC CGCGCCAGAG CGTATGCAAG ACCCTGGCGC 2260

GGTTGGCCGG TGATCGTTCA ATAGTGCGAA TATGAATGGT TACCAGCCGC CTGCGAATTC 2320
**(2) INFORMATION FOR SEQUENCE IDENTIFICATION NUMBER:** 2:
   **(i) SEQUENCE CHARACTERISTICS:**
      **(A) LENGTH:** 53
      **(B) TYPE:** nucleic acid
      **(C) STRANDEDNESS:** single
      **(D) TOPOLOGY:** linear
   **(xi) SEQUENCE DESCRIPTION:** SEQ ID NO: 2: CATTTCTCAT TGATAATGAG AATCATTATT GACATAATTG TTATTATTTT ACG 53
**(2) INFORMATION FOR SEQUENCE IDENTIFICATION NUMBER:** 3:
   **(i) SEQUENCE CHARACTERISTICS:**
      **(A) LENGTH:** 688
      **(B) TYPE:** nucleic acid
      **(C) STRANDEDNESS:** double
      **(D) TOPOLOGY:** linear
   **(xi) SEQUENCE DESCRIPTION: SEQ ID NO:** 3: 
GAGCGCATTA TCAGATAAAT TGATTTATTT CTCACTTTCA TTCTATTTTC ATCAGGAATC 60
CCTGTGTCCT GTGCGGTAAT CTGCTGCTAT CGAGAACGAC AGACATCGCT AACAGTATAT 120
ATGGAAACAT CAAAAGAGAA GACGATAACA AGCCCAGGGC CATACATAGT TCGATTACTT 180
AACAGCTCAC TGAACGGCTG TGAGTTTCCA TTGCTGACAG GCCGAACACT CTTTGTGGTA 240
GGTCAGAGTG ATGCGCTCAC TGCTTCAGGT CAACTCCCTG ATATACCTGC CGATAGCTTT 300
TTTATCCCGC TGGACCATGG CGGAGTAAAT TTTGAAATCC AGGTGGATAC GGATGCGACC 360
GAAATTATAC TCCATGAGCT GAAAGAAGGA AATTCTGAAT CTCGTTCGGT GCAATTAAAT 420
ACGCCAATAC AGGTCGGTGA ATTGCTTATC CTGATTCGCC CGGAAAGCGA GCCGTGGGTG 480
CCCGAGCAGC CTGAGAAGTT AGAAACGTCT GCAAAAAAGA ACGAGCCGCG TTTTAAAAAC 540
GGAATTGTAG CAGCACTGGC CGGGTTTTTT ATATTGGGAA TTGGGACTGT GGGGACGTTA 600
TGGATACTTA ACTCGCCGCA GCGGCAGGCC CGAGAGCTCG ATTCGTTATT GGGGCAGGAG 660
AAGGAGCGTT TTCAGGTGTT GCCAGGCC 688
**(2) INFORMATION FOR SEQUENCE IDENTIFICATION NUMBER:** 4:
   **(i) SEQUENCE CHARACTERISTICS:**
      **(A) LENGTH:** 16
      **(B) TYPE:** nucleic acid
      **(C) STRANDEDNESS:** single
      **(D) TOPOLOGY:** linear
   **(ii) MOLECULE TYPE:** DNA (genomic)
   **(xi) SEQUENCE DESCRIPTION: SEQ ID NO:** 4: AATATCGCCC TGAGCA 16
**(2) INFORMATION FOR SEQUENCE IDENTIFICATION NUMBER:** 5:
   **(i) SEQUENCE CHARACTERISTICS:**
      **(A) LENGTH:** 4044
      **(B)** TYPE: nucleic acid
      **(C) STRANDEDNESS:** double
      **(D) TOPOLOGY:** linear
   **(ii) MOLECULE TYPE:** DNA (genomic)
   **(xi) SEQUENCE DESCRIPTION: SEQ ID NO:** 5: 
GGTTAACTCT TCGTTGAATA AAAAATGTCA ATGACGTTCC ATAATTCAGG AGATGAACTT 60
CACAAGTCAT TATATATAAC AGGAGGTGCT ATGAAACATC ATGCTTTTAT GCTTTGGTCA 120
TTACTTATTT TTTCATTCCA TGTTTTGGCC AGTTCAGGCC ATTGTTCTGG TTTACAACAG 180
GCATCATGGG ATATTTTTAT CTACGATTTT GGTAGTAAAA CCCCGCAACC ACCTACAAAT 240
ACTGATAAAA AGCAAGCCAG GCAGATTAGT TCACCGTCCT GCCCGACGAC AAAACCCATG 300
ATGTCCGCAC CAGTCAATGA CGCCAGGAAA GGGAATACTT TCTCCAGAAC ATAATGTTAT 360
TTATCTACAA TGGTGCCGAC GACTACTTTT AGCCACCCGG AAATCTTGAT TGCCATCAAA 420
TATAGCTGGC ATTATTTTTC CTGACGTGTA TAGTGCGCCT CGTTATCCCC ATTAAGGAAT 480
TTGTTTGTCT CGTAAAATGA CAGGAATTGT CAAAACCTTT GATTGTAAGA GCGGTAAAGG 540
TCTCATCACC CCCTCCGATG ACGCAAAGAT GTTCAGGTCC ACATTTCAGC ATGTCGCCAA 600
CACGAAACAG AAGCGCTTAT CCCCGGTATA CGCGTTGAGT TTTATCGTAT TAATGGCCTC 660
CGCGGACCTA CCGCCGCCAA CGTTTATCTT TCATAATTCG TCACCCGGCA TTTTTCAGAA 720
AAATTTAGCG AGTACGTCTA CCTCCGCAGC CTGCTATGAG GCTTTGCCTG AAAGGCTGCA 780
GAATGTTTTC AGTGGCGAAA ATCTAAAAGA TTTATTTTGC TAATCAGTCC TGTGACCTCT 840
 
TTTATCATAT ATCGGGTGCC CCCCCTTCTC ACTTTGTTTA ACGTGAAGAA ATGTACAGCC 900
GTTTTTCACT GTGATAGCAT CTAATATTGC AAAAGTATTT AACGCTATAT ACCCATTGTC 960
ACAGGAGTGG CTGCGTGCGA GCTGAGCTAT TTAACCGAAG TATTTATGTG ATCATTGGAA 1020
TTATCTCTAT TGCCGCTCAA TGCTACGTCA TATTCAGTGG GTATAAATCG CCAATATAGT 1080
TGTAACGCTA TTTATTTTTA GGGTAATAAT TGAATGACTT TGCTTTCAGG AAAAACCACA 1140
CTGGTTCTCT GCCTCTCCTC TATTTTATGT GGATGTACGA CGAACGGCTT ACCCACACCT 1200
TATAGTATTA ATTTGTCGTT CCCGGTCATT ACACAAAACC AGATTAATTC CGGTGGTTAT 1260
TACATAAATG ACGCGGAACA AATTCGGACA ACTGATGGTC TGTGCCTTGA TGCAGGCCCA 1320
GATCAACAGA ATCGTTTGAC GCTGCGGGAG TGTAAGCATG TGCAATCTCA GCTTTTCTCA 1380
TTTCACCGAG ACAGAATCAC GCAGGGTGAG AAATGTCTGG ATGCCGCAGA CAAGGTACAA 1440
AAGAAGGCAC ACCAATCATT CTTTATTCAT GCACGGGTAA TGATAACCAG CGCTGGCTCA 1500
CTGATCATAA CAAAATTAAG GGGAAACAGA GCCGAAAATG CCTGGGCACA AATAGCATTA 1560
TTGTCAGAAA AGGCGACCCT GTTGTGTTGG CCGATTGCGA TTTTAGTCGC GCCCTGGAAT 1620
TTACCATCAG GTAGCAGGAC ACCGCTGTGA AGAGAGTGCC GCTAACCTCT TGACACGACA 1680
ACAGGTTAGC GACCTTTACT TCCACGTGCG ATCAATTTAC TTTACGTCCG CAACGTCAGG 1740
ATGACAAAAC GGCGGCTAAA CCTTGACACC AGTTATATAC CCAGCTTAAA TACTGGTCAT 1800
CCAACCAGTA AAAAGGAAAT GGCGATGTTC GTCGAACTCG TTTATGACAA GCGAAATGTT 1860
GAAGGTTTGC CAGGCGCACG CGAAATCATC CTCAATGAAC TCACAAAACG CGTACATCAA 1920
CTTTTTCCCG ATGCGCAAGT GAAAGTTAAG CCAATGCAGG CGAACGCATT AAACAGTGAC 1980
TGTACAAAAA CCGAGAAAGA ACGGCTGCAC CGTATGCTGG AAGAGATGTT TGAAGAGGCT 2040
GATATGTGGC TGGTCGCCGA ATAACGTCCC CTCCTGCGAA AGCCAACATG TCCGATCGAA 2100
AACAGCGCCC TGAGGCGCTG TCTGTGACGA TATAACGCAA ACGCTACCAC TCAGAACATG 2160
TTGTTGTTGA TACCTCAGAC CGGTATGTGG AACCGACATT CATCGCTTCA CTGGCCTGTC 2220
GGTATGAGTA GCCCTTATCA ACAATCAGCT GTGCGCATTC CAGCCTGAAA TCTGAAAGTA 2280
CGTTTGGTTT TGTTGTTTAT TAAGAGCCTA TCCCATTAGA CTCTTTTATT CGCCAAACTG 2340
GCTTTAACGA TTACGCCTAC TGGGATAGGT TCTAAACTTA TCATCAATAC GTAAAATACC 2400
TATTTACGAA CAAAAAGTAA CAGGTAAAAA TCCGAAATAA AACCAGCATA ACTAAAACTT 2460
ACTGCAGATA TGCACACGCA TTATTACTAT GTTTCCAGGA TAGTCTCGAC CAGTCAAGAC 2520
TATCTATTTT ATATAAAAAG GGAAATACTT CACATGAATA AAATACATGT TACATATAAA 2580
AATCTCTTAC TTCCGATTAC CTTCATCGCG GCAACTCTAA TTAGCGCCTG TGATAACGAT 2640
AAAGATGCCA TGGCGGAAGC TGAAAAAAAT CAAGAGAAAT ACATGCAAAA AATCCAGCAA 2700
AAAGAGCACC AGCAATCAAT GTTCTTTTAC GACAAAGCCG AAATGCAAAA AGCTATTGCC 2760
 
AATATCAACG CAAAAGGTGG AGCCAATCTT GCGATTATTG AAGTCCGTTT CTTCAAGGGC 2820
GGGTATTCAT TCATTCGACA AAGCGTTAAC ACCCCTGCTA AAGTAGAGGT GTTTAAATTT 2880
AACAACGGCT ACTGGGGGGG ACCTTCGCCT GTCAATTTAA CCATCTTTGG CACTATAACA 2940
GAGGAGCAAA AACAAGAAGC ACTAAAAGAG GCTTTATTCA AATTCGACTC GATCAATTTC 3000
AGCATTATAC CAGAGCGTAT TCAGGAAACA ATTAAACGCG CTAACGCCAG TGGCATCATT 3060
TCCGTTACGG AAGATAGCGA TATCGTTGTA CGAGCAGAGA TAGCTCATAA TGGCGAATTC 3120
GTCTATGACA TTACCATCAC TGCTAAAAAT ACAGCACGTG CGGTAATGAC CTTAAATAAG 3180
GATGGTTCTA TTGCCGGATA TGAGATCAAA GAACCTTTCG CCCCAAAAAA AGAAGCCGAA 3240
AAAGCACAGC AACTTGTTGA ACAATCGAGA AAAGACATTG AAAGTCCAGC GTAAAAAAGC 3300
AGCTGGAAAG ATGAACGAAA TACAGCAGAC ATTTAAAAAT AGCAGGCGAT ACAAACATTG 3360
ATAAAAATTA TAGCGCGAAA GAGCGCGTGC CAGGTACTAA GGCACTGCTT GAAGACAGCG 3420
AATCGCTATT TCATTCTCTG ACACTGTAAT TTTTCGTACT CAAGATGTTT ATTTATTGAG 3480
TCTTTTGTGG ATAACCAGGT GAAGTTATGT GACGCCAGGA ATCTATTCCA GCGGGCGTAC 3540
TTGTTGGAGC CAGTGTGAAG CCGGGCAGCG CGCAGAAACC GGAGCGTATA CGTTGTACGT 3600
AAGAATTTCG AGCACTGCCC GACCTAAAAA TGATGAATAA AATAGATATT TTAAAGAGGT 3660
AATATGAAGA ATTTTTTCAA AATAATTACT GATTTCATCG CGGATATTTC CCTTGATCTA 3720
TTTGCTATAT TTTTATGCAT GTTATTCGTA TACAAAACAG GACCATCAAT TGGTGTGATA 3780
TCATTTTTTA TTGCATTAAT TATTTATATC ATTCTTCATT TTTTTTTACT CATTTCTTGA 3840
AAAAATCATA AAAAAAATAT TCAAATAAGT ATTTAAAATT ATTGTTTTGT GGTACAAATT 3900
CAGCGCAATA AAACAGAGCA ACTAAAAAAA ATTAGGCGTA GCGAAGTGGA AAAGGACTGT 3960
CATGTACTGG ACCGTGAGCT GGTCGGGAGA GCAATGTACG GGAAAGAGCG AAATACTGTC 4020
ATTGATATGA GCAGGAATAT CGAT 4044
**(2) INFORMATION FOR SEQUENCE IDENTIFICATION NUMBER: 6:**
   **(i) SEQUENCE CHARACTERISTICS:**

   **(i) SEQUENCE CHARACTERISTICS:**
      **(A) LENGTH:** 87 amino acids
      **(B) TYPE:** amino acid
      **(D) TOPOLOGY:** linear
   **(ii) MOLECULE TYPE:** peptide
   **(xi) SEQUENCE DESCRIPTION:** SEQ ID NO: 6: 
Met Lys His His Ala Phe Met Leu Trp Ser Leu Leu Ile Phe Ser Phe
1               5                   10                  15
His Val Leu Ala Ser Ser Gly His Cys Ser Gly Leu Gln Gln Ala Ser
            20                  25                  30
Trp Asp Ile Phe Ile Tyr Asp Phe Gly Ser Lys Thr Pro Gln Pro Pro
        35                  40                  45
Thr Asn Thr Asp Lys Lys Gln Ala Arg Gln Ile Ser Ser Pro Ser Cys
    50                  55                  60
Pro Thr Thr Lys Pro Met Met Ser Ala Pro Val Asn Asp Ala Arg Lys
65                  70                  75                  80
Gly Asn Thr Phe Ser Arg Thr
                85
**(2) INFORMATION FOR SEQUENCE IDENTIFICATION NUMBER:** 7:
   **(i) SEQUENCE CHARACTERISTICS:**
      **(A) LENGTH:** 178 amino acids
      **(B) TYPE:** amino acid
      **(D) TOPOLOGY:** linear
   **(ii) MOLECULE TYPE:** peptide
   **(xi) SEQUENCE DESCRIPTION: SEQ ID NO:** 7 
Met Thr Leu Leu Ser Gly Lys Thr Thr Leu Val Leu Cys Leu Ser Ser
1               5                   10                  15

Ile Leu Cys Gly Cys Thr Thr Asn Gly Leu Pro Thr Pro Tyr Ser Ile
            20                  25                  30

Asn Leu Ser Phe Pro Val Ile Thr Gln Asn Gln Ile Asn Ser Gly Gly
        35                  40                  45

Tyr Tyr Ile Asn Asp Ala Glu Gln Ile Arg Thr Thr Asp Gly Leu Cys
    50                  55                  60

Leu Asp Ala Gly Pro Asp Gln Gln Asn Arg Leu Thr Leu Arg Glu Cys
65                  70                  75                  80

Lys His Val Gln Ser Gln Leu Phe Ser Phe His Arg Asp Arg Ile Thr
                85                  90                  95

Gln Gly Glu Lys Cys Leu Asp Ala Ala Asp Lys Val Gln Lys Lys Ala
            100                 105                 110

His Gln Ser Phe Phe Ile His Ala Arg Val Met Ile Thr Ser Ala Gly
        115                 120                 125

Ser Leu Ile Ile Thr Lys Leu Arg Gly Asn Arg Ala Glu Asn Ala Trp
    130                 135                 140

Ala Gln Ile Ala Leu Leu Ser Glu Lys Ala Thr Leu Leu Cys Trp Pro
145                 150                 155                 160
 
Ile Ala Ile Leu Val Ala Pro Trp Asn Leu Pro Ser Gly Ser Arg Thr
                165                 170                 175

Pro Leu
**(2) INFORMATION FOR SEQUENCE IDENTIFICATION NUMBER:** 8:
   **(i) SEQUENCE CHARACTERISTICS:**
      **(A) LENGTH:** 79 amino acids
      **(B) TYPE:** amino acid
      **(D) TOPOLOGY:** linear
   **(ii) MOLECULE TYPE:** peptide
   **(xi) SEQUENCE DESCRIPTION: SEQ ID NO:** 8 
Met Phe Val Glu Leu Val Tyr Asp Lys Arg Asn Val Glu Gly Leu Pro
1               5                   10                  15

Gly Ala Arg Glu Ile Ile Leu Asn Glu Leu Thr Lys Arg Val His Gln
            20                  25                  30

Leu Phe Pro Asp Ala Gln Val Lys Val Lys Pro Met Gln Ala Asn Ala
        35                  40                  45

Leu Asn Ser Asp Cys Thr Lys Thr Glu Lys Glu Arg Leu His Arg Met
    50                  55                  60

Leu Glu Glu Met Phe Glu Glu Ala Asp Met Trp Leu Val Ala Glu
65                  70                  75
**(2) INFORMATION FOR SEQUENCE IDENTIFICATION NUMBER:** 9:
   **(i) SEQUENCE CHARACTERISTICS:**
      **(A) LENGTH:** 246 amino acids
      **(B) TYPE:** amino acid
      **(D) TOPOLOGY:** linear
   **(ii) MOLECULE TYPE:** peptide
   **(xi) SEQUENCE DESCRIPTION:** SEQ ID NO: 9 
Met Asn Lys Ile His Val Thr Tyr Lys Asn Leu Leu Leu Pro Ile Thr
1               5                   10                  15

Phe Ile Ala Ala Thr Leu Ile Ser Ala Cys Asp Asn Asp Lys Asp Ala
            20                  25                  30

Met Ala Glu Ala Glu Lys Asn Gln Glu Lys Tyr Met Gln Lys Ile Gln
        35                  40                  45
 
Gln Lys Glu His Gln Gln Ser Met Phe Phe Tyr Asp Lys Ala Glu Met
    50                  55                  60

Gln Lys Ala Ile Ala Asn Ile Asn Ala Lys Gly Gly Ala Asn Leu Ala
65                  70                  75                  80

Ile Ile Glu Val Arg Phe Phe Lys Gly Gly Tyr Ser Phe Ile Arg Gln
                85                  90                  95

Ser Val Asn Thr Pro Ala Lys Val Glu Val Phe Lys Phe Asn Asn Gly
            100                 105                 110

Tyr Trp Gly Gly Pro Ser Pro Val Asn Leu Thr Ile Phe Gly Thr Ile
        115                 120                 125

Thr Glu Glu Gln Lys Gln Glu Ala Leu Lys Glu Ala Leu Phe Lys Phe
    130                 135                 140

Asp Ser Ile Asn Phe Ser Ile Ile Pro Glu Arg Ile Gln Glu Thr Ile
145                 150                 155                 160

Lys Arg Ala Asn Ala Ser Gly Ile Ile Ser Val Thr Glu Asp Ser Asp
                165                 170                 175

Ile Val Val Arg Ala Glu Ile Ala His Asn Gly Glu Phe Val Tyr Asp
            180                 185                 190

Ile Thr Ile Thr Ala Lys Asn Thr Ala Arg Ala Val Met Thr Leu Asn
        195                 200                 205

Lys Asp Gly Ser Ile Ala Gly Tyr Glu Ile Lys Glu Pro Phe Ala Pro
    210                 215                 220

Lys Lys Glu Ala Glu Lys Ala Gln Gln Leu Val Glu Gln Ser Arg Lys
225                 230                 235                 240

Asp Ile Glu Ser Pro Ala
                245
**(2) INFORMATION FOR SEQUENCE IDENTIFICATION NUMBER: 10:**
   **(i) SEQUENCE CHARACTERISTICS:**
      **(A) LENGTH:** 3700
      **(B) TYPE:** nucleic acid
      **(C) STRANDEDNESS:** double
      **(D) TOPOLOGY:** linear
   **(ii) MOLECULE TYPE:** DNA (genomic)
   **(xi) SEQUENCE DESCRIPTION: SEQ ID NO:** 10 
TTTTGGTTTG CTGCCGTTTG GGATAACTGC ATAGAGAGCG GCCAAGTCGC TTGCGGTCGG 60
TATCTCGAGT ATATCGAAAT CCATGTGGCC ATTGACCTCT TCAAGCGCTC ACGTTAACTA 120
CCTGCTCTTT TTTGAGCACC AACATCCCAG GTTCGTCACA GTAAATCGTA TCGTGATTAT 180
 
TGCTAATCGT CAGTTTACCG CTCCGAAAGC AAACTAAAGT GAAACTGCTT ACATAAAGAT 240
TTTTGATGGT AACCTGCTGA GTCTGACTTT TAATTTGCTG CCGGGTATTT GTCAAAAGTG 300
ATTTTAATTT CTGTAAGTTA TCTGCGGCAG GACGCTGATG ACTATTACTT ACAAAGGTTA 360
CATTTTCCAT ATTATCCCTT TGTTGAACTT ATTTTAATGT TCCTTACTGG TATCCTACTG 420
AAAAAATCTG AGTTGTAAAT GCTCTTTATT AGCGTGTGTT GGCAATGGTC TGATTGTTAC 480
ACCAAAAGAA CCCAAATTTG GGTAATTTAT CTACAGTAGT TTAAGCCCCA ATGGGGATGA 540
TGGTTCTTTT AATATGTGTT GAGACGCATT ATACAGAATA AATTGATTTT ATTTCTCACT 600
TTTCATTCTA TTTTCATCAG GAATCCCTGT GTCCTGTGCG GTAATCTGCT GCTATCGAGG 660
AACGACAGAC ATCGCTAACA GTATATATGG AAACATCAAA AGAGAAGACG ATAACAAGCT 720
TTCCAGGGCC ATACATAGTT CGATTACTTA ACAGCTCACT GAACGGCTGT GAGTTTCCAT 780
TGGGCCTGAC AGGCCGAACA CTCTTTGTGG TAGGTCAGAG TGATGCGCTC ACTGCTTCAG 840
GTCAATGTGA TAGCTCCCTG ATATACCTGC CGATAGCTTT TTTATCCCGC TGGACCATGG 900
CGGAGTAAAT TTTAGGGAAA TCCAGGTGGA TACGGATGCG ACCGAAATTA TACTCCATGA 960
GCTGAAAGAA GGAAATTATG TCTGAATCTC GTTCGGTGCA ATTAAATACG CCAATACAGG 1020
TCGGTGAATT GCTTATCCTG TGATTCGCCC GGAAAGCGAG CCGTGGGTGC CCGAGCAGCC 1080
TGAGAAGTTA GAAACGTCTG CATAAAAAAG AACGAGCCGC GTTTTAAAAA CGGAATTGTA 1140
GCAGCACTGG CCGGGTTTTT TATAGAAAGT TGGGAATTGG GACTGTGGGG ACGTTATGGA 1200
TACTTAACTC GCCGCAGCGG CAGGCCGCAG GTGTAAGAGC TCGATTCGTT ATTGGGGCAG 1260
GAGAAGGAGC GTTTTCAGGT GTTGCCAGGC CGGGACGGAA AATGCTCTAT GTCGCTGCGC 1320
AAAATGAAAG AGATACGTTG TGGGCTCGTC AGGTTTTAAA TAGCGAGGGG CGATTATGAT 1380
AAAAATGCGC GAGTGATTAA CGAAAACGAA GAAAATAAGC GTAGAATCTC TATCTGGCTG 1440
GATACCTATT ATCCGCAGCT GGCTTATTAT CGGATTCATT TCGATTAGAG CCGCGTAAAC 1500
CCGTTTTCTG GCTAAGCCGC CAGCGAAACA CGATGAGCAA GAAAGAGTCT CGAGGTGTTA 1560
AGTCAAAAGC TGAGAGCGCT AATGCCTTAC GCGGATTCGG TTAACATCAA ACGTTGATGG 1620
ACGATGTTAC CCCAGCAGGC CAGGCGGAAG CGGGGCTAAA ACAGCAGGCG TTAAGAAGAT 1680
TACCTTATTC CCGCAGGAAT CATAAGGGGG GCGTAACGTT TGTTATTCAG GGGGCGCTCG 1740
GTGAGATGAT GTAGAAATAC TCAGAGCCCG TCAATTTGTC GATAGCTATT ACCGCACATG 1800
GGGAATGGGA CGCTATGTGC AGTTTGCGAT CGAATTAAAA GATGACTGGC TCAAGGGGCG 1860
CTCATTTGAG CAGTACGGGG CGGAAGGTTA TATCAAAATG AGCCCAGGCC ATTGGTATTT 1920
CCCAAGCCCA GAGGGCTTTA ATTTAACGTA AATAAGGAAG TCATTATGGC AACACCTTGG 1980
TCAGGCTATC TGGATATGGA CGTCTCAGCA AAATTTGATA CGGGCGTTGA TAATCTACAA 2040
ACGCAGGTAA CAGAGGCGAT GTTACTGGAT AAATTAGCAG CAAAACCCTC CGATCCGGCG 2100
 
CTACTGGCGG CGTATCAGAG TAAGAAAAAC TCTCGGAATA TAACTTGTAC CGTAACGCGC 2160
AATCGAACAC GGTAAAAGTC TTTAAGGATA TGATTGATGC TGCCATTATT CAGAACTTCC 2220
GTTAATCAGT TATAAGGTGG ATTATGTCGA TTAAGCAACT ATTGTCCCTG AGAATGCCGT 2280
TATAGGGCAG GCGGTCAATA TCAGGTCTAT GGAAATAGAA CGGACATTGT CTCGCTGGAT 2340
GACCGGCTAC TCCAGGCTTT TTCTGGTTCG GCGATTGCCT AGAAACGGCT GTGGATAAAC 2400
AGACGATTAC CAACAGGATT GAGGACCCTA ATCTGGTGAC GGATTATTTC CTAAAGAGCT 2460
GGCTATTTCG CAAGAGATGA TTTCAGATTA TAACCTGTAT GTTTCTATGA GGTCAGTACC 2520
CTTACTCGTA AAGGAGTCGG GGCTGTTGAA ACGCTATTAC GCTCATGATT CTTGGATGTC 2580
GATATCTATA TACTTTTCTG CTGGTAATGA CCCTTGCCGG CTGTAAGGAT AAGGATCTTA 2640
GCTTTTAAAA GGACTGGACC AGGAACAGGC TAATGAGGTC ATTGCCGTTC TGCAAATGCA 2700
CAGAAATATA GAGGCGAATA AAATTGATAG CGGAAAATTG GGCTATAGCA TTACCGTTGC 2760
TGAGCAGGTA CTGATTTTAC CGCTGCGGTG TACTGGATTA AAACTTATCA GCTTCCTCCC 2820
CGGCCACGGG TAATTGGAAA TAGCGCAGAT GTTCCCGGCG GATTCGCTGG TATCGTCTCC 2880
GCGAGCTGAA AAGGAAAACC AGGTTATATT CGGCTATTGA ACAGCGACTG GAACAGTCAT 2940
TACAGACGAT GGAGGGCGAT GTGCTCTCCG CCAGGGTCCA TATTAGTTAT GATATTGATG 3000
CTGGTGAAAA TGGCCGCCCG CAAGGCAAAA CCTGTTCATC TGTCGGCATT AGCCGTATAT 3060
GAACGAGGTT CGCCGCTTGC GCATCAAGAA GATCAGCGAT ATCAAGCGTT TCTTAAAGAA 3120
TAGTTTTGCC GATGTGGATT ATGACAACAA TTTCTGTTGT GTTGTCAGAA CGTTCTGATG 3180
CCCAATTACA GGCTCCCGGC ACACCAGTAA AAGTAACGTA ATTCTTTTGC AACCAGTTGG 3240
ATTGTTTTGA TTATTTTGTT ATCCGTGATG TCAGATACAG GCTTTGGCGT CTGGTATTAC 3300
AAAAACCATT ATGCCCGCAA TAAGAAAGGC ATAACGGGGA GTACTGATGA TAAGGCGAAA 3360
TCGTCAAATG AATAGGCAGC CATTACCCAT TATCTGGCAA AGAATCATTT TTGATCCGTT 3420
ATCGTATATC CATCCTCAGC GGTTGCAGAT AGCGCCGGAA ATGATTGTCA GACCGCGCCA 3480
CGCGAAATGA GTTAATACTG GCGGCATGGC GGCGGCTTAA GAACGGAGAA AAGGAGTGTA 3540
TTCAAAACTC ACTGACGCAG CTGTGGCTGC TCAGTGGCGC CGACTGCCGC AAGTAGCGTA 3600
TTTACTAAAC TGAGAGCCGA TCTGGCAAGG CAGGGAGCCT TGCTTGGCCT AGCCGGATTG 3660
GGCGAAATGA GTTAATACTG GCGGCATGGC GGCTTGCCAT 3700
**(2) INFORMATION FOR SEQUENCE IDENTIFICATION NUMBER:** 11:
   **(i) SEQUENCE CHARACTERISTICS:**
      **(A) LENGTH:** 392 amino acids
      **(B) TYPE:** amino acid
      **(D) TOPOLOGY:** linear
   **(ii) MOLECULE TYPE:** peptide
   **(xi) SEQUENCE DESCRIPTION:** SEQ ID NO: 11 
Met Glu Thr Ser Lys Glu Lys Thr Ile Thr Ser Pro Gly Pro Tyr Ile
1               5                   10                  15

Val Arg Leu Leu Asn Ser Ser Leu Asn Gly Cys Glu Phe Pro Leu Leu
            20                  25                  30

Thr Gly Arg Thr Leu Phe Val Val Gly Gln Ser Asp Ala Leu Thr Ala
        35                  40                  45

Ser Gly Gln Leu Pro Asp Ile Pro Ala Asp Ser Phe Phe Ile Pro Leu
    50                  55                  60

Asp His Gly Gly Val Asn Phe Glu Ile Gln Val Asp Thr Asp Ala Thr
65                  70                  75                  80

Glu Ile Ile Leu His Glu Leu Lys Glu Gly Asn Ser Glu Ser Arg Ser
                85                  90                  95

Val Gln Leu Asn Thr Pro Ile Gln Val Gly Glu Leu Leu Ile Leu Ile
            100                 105                 110

Arg Pro Glu Ser Glu Pro Trp Val Pro Glu Gln Pro Glu Lys Leu Glu
        115                 120                 125

Thr Ser Ala Lys Lys Asn Glu Pro Arg Phe Lys Asn Gly Ile Val Ala
    130                 135                 140

Ala Leu Ala Gly Phe Phe Ile Leu Gly Ile Gly Thr Val Gly Thr Leu
145                 150                 155                 160

Trp Ile Leu Asn Ser Pro Gln Arg Gln Ala Ala Glu Leu Asp Ser Leu
                165                 170                 175

Leu Gly Gln Glu Lys Glu Arg Phe Gln Val Leu Pro Gly Arg Asp Lys
            180                 185                 190

Met Leu Tyr Val Ala Ala Gln Asn Glu Arg Asp Thr Leu Trp Ala Arg
        195                 200                 205

Gln Val Leu Ala Arg Gly Asp Tyr Asp Lys Asn Ala Arg Val Ile Asn
    210                 215                 220

Glu Asn Glu Glu Asn Lys Arg Ile Ser Ile Trp Leu Asp Thr Tyr Tyr
225                 230                 235                 240

Pro Gln Leu Ala Tyr Tyr Arg Ile His Phe Asp Glu Pro Arg Lys Pro
                245                 250                 255

Val Phe Trp Leu Ser Arg Gln Arg Asn Thr Met Ser Lys Lys Glu Leu
            260                 265                 270

Glu Val Leu Ser Gln Lys Leu Arg Ala Leu Met Pro Tyr Ala Asp Ser
        275                 280                 285

Val Asn Ile Thr Leu Met Asp Asp Val Thr Ala Ala Gly Gln Ala Glu
    290                 295                 300
 
Ala Gly Leu Lys Gln Gln Ala Leu Pro Tyr Ser Arg Arg Asn His Lys
305                 310                 315                 320

Gly Gly Val Thr Phe Val Ile Gln Gly Ala Leu Asp Asp Val Glu Ile
                325                 330                 335

Leu Arg Ala Arg Gln Phe Val Asp Ser Tyr Tyr Arg Thr Trp Gly Gly
            340                 345                 350

Arg Tyr Val Gln Phe Ala Ile Glu Leu Lys Asp Asp Trp Leu Lys Gly
        355                 360                 365

Arg Ser Phe Gln Tyr Gly Ala Glu Gly Tyr Ile Lys Met Ser Pro Gly
    370                 375                 380

His Trp Tyr Phe Pro Ser Pro Leu
385                 390
**(2) INFORMATION FOR SEQUENCE IDENTIFICATION NUMBER:** 12:
   **(i) SEQUENCE CHARACTERISTICS:**
      **(A) LENGTH:** 80 amino acids
      **(B) TYPE:** amino acid
      **(D) TOPOLOGY:** linear
   **(ii) MOLECULE TYPE:** peptide
   **(xi) SEQUENCE DESCRIPTION: SEQ ID NO:** 12 
Met Ala Thr Pro Trp Ser Gly Tyr Leu Asp Asp Val Ser Ala Lys Phe
1               5                   10                  15
Asp Thr Gly Val Asp Asn Leu Gln Thr Gln Val Thr Glu Ala Leu Asp
            20                  25                  30
Lys Leu Ala Ala Lys Pro Ser Asp Pro Ala Leu Leu Ala Ala Tyr Gln
        35                  40                  45
Ser Lys Leu Ser Glu Tyr Asn Leu Tyr Arg Asn Ala Gln Ser Asn Thr
    50                  55                  60
Val Lys Val Phe Lys Asp Ile Asp Ala Ala Ile Ile Gln Asn Phe Arg
65                  70                  75                  80
**(2) INFORMATION FOR SEQUENCE IDENTIFICATION NUMBER:** 13:
   **(i) SEQUENCE CHARACTERISTICS:**
      **(A) LENGTH:** 101 amino acids
      **(B) TYPE:** amino acid
      **(D) TOPOLOGY:** linear
   **(ii) MOLECULE TYPE:** peptide
   **(xi) SEQUENCE DESCRIPTION:** SEQ ID NO: 13 
Met Ser Ile Ala Thr Ile Val Pro Glu Asn Ala Val Ile Gly Gln Ala
1               5                   10                  15
Val Asn Ile Arg Ser Met Glu Thr Asp Ile Val Ser Leu Asp Asp Arg
            20                  25                  30
Leu Leu Gln Ala Phe Ser Gly Ser Ala Ile Ala Thr Ala Val Asp Lys
        35                  40                  45
Gln Thr Ile Thr Asn Arg Ile Glu Asp Pro Asn Leu Val Thr Asp Pro
    50                  55                  60
Lys Glu Leu Ala Ile Ser Gln Glu Met Ile Ser Asp Tyr Asn Leu Tyr
65                  70                  75                  80
Val Ser Met Val Ser Thr Leu Thr Arg Lys Gly Val Gly Ala Val Glu
                85                  90                  95
Thr Leu Leu Arg Ser
            100
**(2) INFORMATION FOR SEQUENCE IDENTIFICATION NUMBER:** 14:
   **(i) SEQUENCE CHARACTERISTICS:**
      **(A) LENGTH:** 252 amino acids
      **(B) TYPE:** amino acid
      **(D) TOPOLOGY:** linear
   **(ii) MOLECULE TYPE:** peptide
   **(xi) SEQUENCE DESCRIPTION: SEQ ID NO:** 14 
Met Ile Arg Arg Tyr Leu Tyr Thr Phe Leu Leu Val Met Thr Leu Ala
1               5                   10                  15
Gly Cys Lys Asp Lys Asp Leu Leu Lys Gly Leu Asp Gln Glu Gln Ala
            20                  25                  30
Asn Glu Val Ile Ala Val Leu Gln Met His Asn Ile Glu Ala Asn Lys
        35                  40                  45
Ile Asp Ser Gly Lys Leu Gly Tyr Ser Ile Thr Val Ala Glu Pro Asp
    50                  55                  60
Phe Thr Ala Ala Val Tyr Trp Ile Lys Thr Tyr Gln Leu Pro Pro Arg
65                  70                  75                  80
Pro Arg Val Glu Ile Ala Gln Met Phe Pro Ala Asp Ser Leu Val Ser
                85                  90                  95
Ser Pro Arg Ala Glu Lys Ala Arg Leu Tyr Ser Ala Ile Glu Gln Arg
            100                 105                 110
 
Leu Glu Gln Ser Leu Gln Thr Met Glu Gly Val Leu Ser Ala Arg Val
        115                 120                 125

His Ile Ser Tyr Asp Ile Asp Ala Gly Glu Asn Gly Arg Pro Pro Lys
    130                 135                 140

Pro Val His Leu Ser Ala Leu Ala Val Tyr Glu Arg Gly Ser Pro Leu
145                 150                 155                 160

Ala His Gln Ile Ser Asp Ile Lys Arg Phe Leu Lys Asn Ser Phe Ala
                165                 170                 175

Asp Val Asp Tyr Asp Asn Ile Ser Val Val Leu Ser Glu Arg Ser Asp
            180                 185                 190

Ala Gln Leu Gln Ala Pro Gly Thr Pro Val Lys Arg Asn Ser Phe Ala
        195                 200                 205

Thr Ser Trp Ile Val Leu Ile Ile Leu Leu Ser Val Met Ser Ala Gly
    210                 215                 220

Phe Gly Val Trp Tyr Tyr Lys Asn His Tyr Ala Arg Asn Lys Lys Gly
225                 230                 235                 240

Ile Thr Ala Asp Asp Lys Ala Lys Ser Ser Asn Glu
                245                 250
(2) INFORMATION FOR SEQUENCE IDENTIFICATION NUMBER: 15:
   **(i) SEQUENCE CHARACTERISTICS:**
      **(A) LENGTH:** 818
      **(B) TYPE:** nucleic acid
      **(C) STRANDEDNESS:** double
      **(D) TOPOLOGY:** linear
   **(xi) SEQUENCE DESCRIPTION:** SEQ ID NO: 15:

CATAACAACT CCTTAATACT ACTTATTATT TACGGTGTGT TTAAACACCT GCAGTACCGA 60
TCCGGCATTC AGTTATCGCC ACTATGCCGA ATCGACAAAA CCACGAATAA TTCACCGCTA 120
TCGCTCCTGA TGTGTTTACT TCCTGAAAGA TATTTTTACT ACCGAAGCAC TCTATCGCTC 180
ATTTAGGTAA CCGGTTCTAC AATGTCATCT AACTTTTATA GATTTGAATG CTAATTTTTC 240
TCACGCATAT ATATTTAACA GAAACCATAA AGTGTTTAGC CACTATAGAA CAACAAATCA 300
CCCATGCAAC ATTTTGATAT TTAAAGAGAA AATCTCACAA CCACATTAAG AAACTTGACA 360
CCGTTCGGCT AAAAAACATG TCATTAAGCA AACTCGCCAT ATAATCAGAA CATATCGCAT 420
TGTGCTTCAC AGTCCTCACG TGACGCTCCA TCCGCAATAC GGTTATATGC CATCGCAGGC 480
GCTGTAATCA TATTCACGAT GATGCTTAGC ACGCTTTATT CCCGCTCCGA TTTAATCTTT 540
TAATATATCT ATCAGTTACA ACATTTCTTG TTATATTATA AGAATAGAAT CAACACCACA 600
ATTCCAACAT AAATATCACC TGTGTTTAGA GAGAATTTAC ATTCCAAAAA AATAATAACT 660

AACGCAAATA TTGAACACGC GATAAAAAAG TCTATTTCGC TATAAAACCC ATTATTATTA 720
AGAGTGGTTA ACTCTTCGTT GAATAAAAAA TGTCAATGAC GTTCCATAAT TCAGGAGATG 780
AACTTCACAA GTCATTATAT ATAACAGGAG GTGCTATG 818

**TABLE 12 Bacterial strains.**

| Strain | Genotype | Source |
|---|---|---|
| *S. typhimurium* | | |
| 14082s | Wild type | ATCC |
| CS019 | *phoN*2 *zxx*::6251Tn*10*d-Cm | 25 |
| CS015 | *phoP*-*102*::Tn*10*d-Cm | 25 |
| AD154 | *phoP12 purB1744*::Tn10 | 3 |
| TT13208 | *phoP105*::Tn*10d* | 26 |
| CS585 | *pagD1*::Tn*phoA phoN2 zxx*::6215Tn*10*d-Cm | This study |
| CS586 | *pagD1*::Tn*phoA phoP105*::Tn*10d phoN2 zxx*::6215Tn*10*d-Cm | This study |
| CS619 | *pogE1*::Tn*phoA phoN2 zxx*::6215Tn*10*d-Cm | This study |
| CS620 | *pagE1*::Tn*phoA phoP105*::Tn*10d phoN2 zxx*::6215Tn*10*d-Cm | This study |
| CS1599 | *pagF1*::*TnphoA pho*N2 *zxx*::6215Tn10d-Cm | This study |
| CS1600 | *pagF1*::Tn*phoA phoP105*::Tn*10*d *phoN2 zxx*::6215Tn*10*d-Cm | This study |
| CS334 | *pagG1*::Tn*phoA phoN*2 *zxx*::6215Tn*10*d-Cm | This study |
| CS335 | *pagG1*::Tn*phoA phoP105*::Tn*10*d *phoN2 zxx*::6215Tn*10*d-Cm | This study |
| CS1488 | *pagH1*::Tn*phoA phoN2 zxx*::6215Tn10d-Cm | This study |
| CS1489 | *pagH1*::Tn*phoA phoP105*::Tn*10*d *phoN2 zxx*::6215Tn*10*d-Cm | This study |
| CS2054 | *pag11*::Tn*phoA phoN2 zxx*::6215Tn*10*d-Cm | This study |
| CS2055 | *pag11*::Tn*phoA phoP105*::Tn*10*d *phoN2 zxx*::6215Tn*10*d-Cm | This study |
| CS1074 | *pagJ1*::Tn*phoA phoN2 zxx*::6215Tn*10*d-Cm | This study |
| CS1075 | *pagJ1*::Tn*phoA phoP105*::Tn*10d phoN2 zxx*::6215Tn*10*d-Cm | This study |
| CS767 | *pagK1*::Tn*phoA phoN2 zxx*::6215Tn10d-Cm | This study |
| CS768 | *pagK1*::Tn*phoA phoP105*::Tn*10d phoN2 zxx*::6215Tn*10*d-Cm | This study |
| CS993 | *pagL1*::Tn*phoA phoN2 zxx*::6215Tn*10*d-Cm | This study |
| CS994 | *pagL1*::Tn*phoA phoP105*::Tn*10d phoN2 zxx*::6215Tn*10*d-Cm | This study |
| CS1845 | *pagM1*::Tn*phoA phoN2 zxx*::6215Tn*10*d-Cm | This study |
| CS1846 | *pagM1*:Tn*phoA phoP105*::Tn*10d phoN2 zxx*::6215Tn*10*d-Cm | This study |
| CS728 | *pagN1*::Tn*phoA phoN2 zxx*::6215Tn*10*d-Cm | This study |
| CS729 | *pagN1*::Tn*phoA phoP105*::Tn*10d phoN2 zxx*::6215Tn*10*d-Cm | This study |
| CS1194 | *pagO1*::Tn*phoA phoN2 zxx*::6215Tn*10*d-Cm | This study |
| CS1195 | *pα8O1::TnphoA phop105::*Tn*10d phoN2* zxxN::6215Tn*10*d-Cm | This study |
| CS1247 | *pagP1::TnphoA phoN2 zxx*::6215Tn*10*d-Cm | This study |
| CS1248 | *pagP1::TnphoA phoP105::*Tn*10d phoN2* zxx::6215Tn*10*d-Cm | This study |
| AK3011-3314 | Collection of Randomly spaced Tn*10* Δ16Δ17 insertions | 18 |
| *E. coli* | | |
| SM10(pRT291) | Contains plasmid pRT291 (TnphoA) derived from pRK290 selecting for Tet'and Km' | 37 |
| MM294(pPH1JI) | Contains Gm' plasmid pPH1JI. which is in incompatible with pRK290 | 37 |

| | | |
|---|---|---|
| 3 Behlau et al., 1993, J. Bacceriol., 175:4475-84 | | |
| 18 Lehrer et al., 1991, Cell, 64:229-30 | | |
| 25 Miller et al., 1989, Proc. Natl. Acad. Sci. USA, 86:5054-58 | | |
| 26 Miller et al., 1990, J. Bacteriol., 172:2485-90 | | |
| 37 Taylor et al., 1989, J. Bacteriol., 171:1870-78 | | |

**TABLE 13 Comparison of pag::phoA activity in strains with wild type and null phoP⁻ loci.**

| Allele | Activity (Units of AP)^{a} | | | | Fold Reduction^{b} |
|---|---|---|---|---|---|
| | Logarithmic growth | | Stationary growth | | |
| | PhoP⁺ | PhoP⁻ | PhoP⁺ | PhoP⁻ | |
| *pagD1::*Tn*phoA* | 32 | 2 | 79 | 9 | 16 |
| *pagE1::*Tn*phoA* | 96 | 2 | 108 | 3 | 48 |
| *pagF1::*Tn*phoA* | 89 | 4 | 276 | 10 | 22 |
| *pagG1::*Tn*phoA* | 35 | 1 | 65 | 6 | 35 |
| *pagH1::*Tn*phoA* | 35 | 5 | 38 | 6 | 7 |
| *pagl1::*Tn*phoA* | 12 | 2 | 24 | 8 | 6 |
| *pagJ1::*Tn*phoA* | 123 | 8 | 944 | 88 | 15 |
| *pagKl::TnphoA* | 30 | 3 | 123 | 26 | 10 |
| *pagL1*::Tn*phoA* | 7 | 1 | 35 | 4 | 7 |
| *pagM1*::*TnphoA* | 92 | 11 | 439 | 130 | 8 |
| *pagN1*::Tn*phoA* | 23 | 1 | 58 | 2 | 23 |
| *pagO1*::Tn*phoA* | 31 | 2 | 54 | 12 | 16 |
| *pagP1*::Tn*phoA* | 38 | 1 | 27 | 3 | 38 |

| | | | | | |
|---|---|---|---|---|---|
| ^{a} The AP activity values are presented in units as defined by Miller for β-galactosidase (24). The values are representative of experiments (performed in duplicate) that were repeated on three separate occasions. PhoP⁺ denotes the *pag*::Tn*phoA* insertion in strain CS019 containing a wild type *phoP* locus. PhoP⁻ denotes an isogenic strains carrying the *phoP105*::Tn*10* allele. | | | | | |
| ^{b} Values of fold reduction in enzymatic activity represent the decrease in AP activity on acquisition of the null *phoP105* allele. These were calculated from logarithmic growth phase cultures and rounded to the nearest whole number. | | | | | |

**TABLE 14 The effects of pag:: phoA gene fusions on Salmonella mouse virulence.**

| Strain | Genotype | LD₅₀ ^{a} | MSI ^{b} | Reference |
|---|---|---|---|---|
| 14028s | Wild type | < 20 | 6.13 | 25 |
| CS015 | *phoP102*::Tn*10-Cam* | 7.0x10⁵ | 0.40 | 25 |
| CS585 | *pagD1*::Tn*phoA* | 4.0x10⁵ | 0.01 | 15 |
| CS1074 | *pagJ1*::Tn*phoA* | 4.0x10³ | 0.56 | This study |
| CS767 | *pagK1*::Tn*phoA* | 9.0x10⁴ | 0.04 | This study |
| CS1845 | *pagM1*::Tn*phoA* | 3.0x10⁴ | 0.09 | This study |

| | | | | |
|---|---|---|---|---|
| ^{a} The 50% lethal dose was determined by intraperitoneal injection of ten mice per dilution using the method of Reed and Muench (31). | | | | |
| ^{b} The Macrophage Survival Index (MSI) was determined by dividing the mean *Salmonella* CFU recovered from macrophage cultures (performed in triplicate) 24 hours after the addition of gentamicin by the mean *Salmonella* CFU recovered from macrophages 1 hour after gentamicin was added. | | | | |
| 16 Kier et al., 1979, J. Bacteriol., 138:155-61 | | | | |
| 25 Miller et al., 1989, Proc. Natl. Acad. Sci. USA, 86:5054-58 | | | | |

**TABLE 15 Plasmids, strains and relevent properties**

| *S. typhimitrium* strains | Relevent genotypes/information | MSI^{a} | Source^{b} |
|---|---|---|---|
| ATCC14028 | Wild type | 3.90 | ATCC |
| CS019 | *phoN2 zxx*::6251Tn*10*d-Cm | | (31) |
| CS585 | CS019, *pagD::TnphoA* | 0.002 | (4) |
| CS586 | CS585; *phoP*105::Tn*10*d-Tet | | (4) |
| JSG205 | ATCC14028, *msgA::Mud*J | 0.01 | This work |
| JSG225 | JSG205, *phoP*105::Tn*10*d-Tet | | This work |
| CS811 | CS019, *envE::TnphoA* | | This work |
| CS812 | CS811, *phoP*105::Tn*10*d-Tet | | This work |
| CS100 | ATCC14028, *phoP*105::Tn*10*d-Tet | 0.01 | derivitive of TT1320 |
| JSG232 | JSG205, *envF*::pGPP2 | | This work |
| JSG234 | CS019, *envF*::pGPP2 | | This work |
| JSG235 | JSG234, *phoP*105::Tn*10*d-Tet | | This work |
| JSG244 | JSG205, *phoP*105::Tn*10*d-Tet | | This work |
| CS099 | ATCC14028;*zxx*3024::Tn*10*Δ16Δ17*pol*-2(Whirfield *polA* amber) | | This work |

| Other salmonellae | | | |
|---|---|---|---|
| Ty2 | Vi positive | | FDA |
| *Salmonella paratyphi* A | ATCC 9150 | | ATCC |
| *Salmonella paratyphi* C | ATCC 13428 | | ATCC |
| *Salmonella enteriditis* | Clinical isolate | | VRI |

| *E*. *coli* Strains | | | |
|---|---|---|---|
| SM10λ*pir* | *thi-1 thr-*1 *leuB6 supE*44 *tonA*21 *lacY*1*recA*::RP4*-2-*Tc::*Mu* | | |
| DH5α | F-Ø 80d*lac*ZΔM15 *Δ(lacZYA-argF)*U16*9endA*1*recA* 1*hsdR*17*deoRthi-*1*supE*44) *gyrA96relA* | | |

| Other *Enterobacteriaceae* | | | |
|---|---|---|---|
| *Yersinia enterocolirica* | Clinical isolate | | MGH bacteriology 1 |
| *Vibrio cholerae* | Clinical isolate | | Peruvian epidermic |
| *Campylobacter fetus* | Clinical isolate | MGH bacteriology lab | |
| *Citrobater freundii* | Clinical isolate | MGH bacteriology lab | |
| *Klebsiella pneunoniae* | Clinical isolate | MGH bacteriology lab | |
| *Shigella flexneri* | Clinical isolate | MGB bacteriology lab | |
| *Shigella sonnei* | Clinical isolate | MGH bacteriology lab | |
| *Morganella morganii* | Clinical isolate | MGH bacteriology lab | |
| *Providencia stuarrii* | Clinical isolate | MGH bacteriology lab | |

| Plasmids | | | |
|---|---|---|---|
| pWPL17 | pBR322 containing a 2.8 Kb *Hpa*I fragment from pWP061 | | This wor |
| pCAA9 | pWPL17 containing a TnphoA insertion in *envF* | | This wor |
| pGP704 | *pir*-dependent suicide vector | | (34) |
| pGPP2 | pGP704 containing the cloned *envF::phoA* gene fusion | | This wor |
| pWP061 | Cosmid clone containing the *pagC* region | | (36) |

| | | | |
|---|---|---|---|
| ^{a} MSI (macrophage survival index) is calculated by dividing the number of surviving organisms at 2 hours post-infection by the number of cell asscociated organisms present after the 30 minute infection | | | |
| ^{b} MGH, Massachusetts General Hospital, ATCC, American Type Culture Collection, FDA, Food a Drug Administration: VRI, Virus Research Institute | | | |
| 4 Belden et al., 1989, Infect. Immun., 57:1-7 | | | |
| 31 Miller et al., 1989, Proc. Natl. Acad. Sci. USA, 86:5054-58 | | | |
| 34 Miller et al., 1988, J. Bacteriol., 170:2575-83 | | | |
| 36 Pulkkinen et al., 1991, J. Bacteriol., 173:86-93 | | | |

**TABLE 16 Alkaline phosphatase and β-galactosidase gene fusion activity**

| Strain | Relevent Genotype | gene fusion activity^{a} |
|---|---|---|
| JSG205 | *msgA*:MudJ | 461(B) |
| JSG244 | *phoP*105::Tn*10*d-Tet *msgA*:MudJ | 415(B) |
| JSG226 | *envE::*Tn*phoA* | 50(A) |
| JSG229 | *phoP*105::Tn*10*d-Tet *envE::*Tn*phoA* | 60(A) |
| JSG204 | *pagD::*Tn*phoA* | 76(A) |
| JSG225 | *phoP*105::Tn*10*d-Tet *pagD::*Tn*phoA* | 9(A) |
| JSG234 | *envF*::pGPP2 | 16(A) |
| JSG235 | *phoP*105::Tn*10*d-Tet *envF*::pGPP2 | 19(A) |
| JSG232 | *msgA*::MudJ *envF*::pGPP2 | 10(A) |

| | | |
|---|---|---|
| ^{a} (A) AP (alkaline phosphatase) or (B) β-gal (β-galactosidase) | | |

## Claims

1. A *Salmonella* cell the virulence of which is attenuated by a non-revertible null mutation within the *phoP*/*phoQ* locus, such that the cell genotype is *phoP*/*phoQ*^{*-*}*,* wherein said non-revertible null mutation comprises a deletion of nucleotides 376-1322 within the *phoP*/*phoQ* locus.

2. The cell of claim 1, wherein said *Salmonella* cell is a *Salmonella typhi* cell, a *Salmonella enteriditis* cell, or a *Salmonella typhimurium* cell.

3. A vaccine comprising a live *Salmonella* cell the virulence of which is attenuated by a non-revertible null mutation within the *phoP*/*phoQ* locus, such that the cell genotype is *phoP*/*phoQ*^{*-*}*,* wherein said non-revertible null mutation comprises a deletion of nucleotides 376-1322 within the *phoP*/*phoQ* locus.

4. The vaccine of claim 3, wherein said *Salmonella* cell is a *Salmonella typhi* cell, a *Salmonella enteriditis* cell, or a *Salmonella typhimurium* cell.

## Revendications

1. Cellule de Salmonella dont la virulence est atténuée par une mutation nulle non réversible dans le locus *phoP*/*phoQ*, de telle sorte que le génotype cellulaire soit *phoP*/*phoQ*^{*-*}, dans laquelle ladite mutation nulle non réversible comprend une délétion des nucléotides 376-1322 dans le locus *phoP*/*phoQ.*

2. Cellule selon la revendication 1, dans laquelle ladite cellule de Salmonella est une cellule de *Salmonella typhi,* une cellule de *Salmonella enteriditis* ou une cellule de *Salmonella typhimurium.*

3. Vaccin comprenant une cellule vivante de Salmonella dont la virulence est atténuée par une mutation nulle non réversible dans le locus *phoP*/*phoQ,* de telle sorte que le génotype cellulaire soit *phoP*/*phoQ*^{*-*}*,* dans lequel ladite mutation nulle non réversible comprend une délétion des nucléotides 376-1322 dans le locus *phoP*/*phoQ.*

4. Vaccin selon la revendication 3, dans lequel ladite cellule de Salmonella est une cellule de *Salmonella typhi,* une cellule de *Salmonella enteriditis* ou une cellule de *Salmonella typhimurium.*

## Patentansprüche

1. *Salmonella-*Zelle*,* deren Virulenz durch eine nicht-reversible Null-Mutation innerhalb des *phoP*/*phoQ-*Lokus attenuiert worden ist, so dass der Zell-Genotyp *phoP*/*phoQ*^{*-*} *ist,* wobei diese nicht-reversible Null-Mutation eine Deletion der Nukleotide 376-1322 innerhalb des *phoP*/*pho*Q-Lokus umfasst.

2. Zelle nach Anspruch 1, wobei diese *Salmonella-*Zelle eine *Salmonella typhi-*Zelle, eine *Salmonella enteriditis*-Zelle oder eine *Salmonella typhimurium*-Zelle ist.

3. Vakzin, umfassend lebende Salmonella-Zellen, deren Virulenz durch eine nicht-reversible Null-Mutation innerhalb des *phoP*/*phoQ-*Lokus attenuiert worden ist, so dass der Zell-Genotyp *phoP*/*phoQ*^{*-*} *ist,* wobei diese nicht-reversible Null-Mutation eine Deletion der Nukleotide 376-1322 innerhalb des *phoP*/*phoQ-*Lokus umfasst.

4. Vakzin nach Anspruch 3, wobei diese *Salmonella-*Zelle eine *Salmonella typhi-*Zelle, eine *Salmonella enteriditis*-Zelle oder eine *Salmonella typhimurium*-Zelle ist.
